(19) Europäisches Patentamt
European Patent Office
Office européen des brevets

(11) EP 3 190 960 B1

(12) EUROPEAN PATENT SPECIFICATION

(45) Date of publication and mention
of the grant of the patent:
**12.01.2022 Bulletin 2022/02**

(51) Int Cl.:
*A61B 5/021* (2006.01)    *A61B 5/02* (2006.01)
*A61B 5/00* (2006.01)

(21) Application number: **15840046.5**

(22) Date of filing: **14.09.2015**

(86) International application number:
**PCT/US2015/050001**

(87) International publication number:
**WO 2016/040947 (17.03.2016 Gazette 2016/11)**

(54) **HYPOVOLEMIA/HYPERVOLEMIA DETECTION USING PERIPHERAL INTRAVENOUS WAVEFORM ANALYSIS (PIVA) AND APPLICATIONS OF SAME**

NACHWEIS VON HYPOVOLÄMIE/HYPERVOLÄMIE MIT PERIPHERER INTRAVENÖSER WELLENFORMANALYSE (PIVA) UND ANWENDUNGEN DAVON

DÉTECTION D'HYPOVOLÉMIE/D'HYPERVOLÉMIE EN UTILISANT UNE ANALYSE DE FORME D'ONDE INTRAVEINEUSE PÉRIPHÉRIQUE (PIVA) ET APPLICATIONS ASSOCIÉE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **12.09.2014 US 201462049829 P**

(43) Date of publication of application:
**19.07.2017 Bulletin 2017/29**

(73) Proprietor: **Vanderbilt University**
**Nashville, TN 37240 (US)**

(72) Inventors:
• **EAGLE, Susan**
**Nashville, Tennessee 37212 (US)**
• **BROPHY, Colleen**
**Nashville, Tennessee 37204 (US)**

• **HOCKING, Kyle**
**Nashville, Tennessee 37205 (US)**
• **BAUDENBACHER, Franz**
**Franklin, Tennessee 37069 (US)**
• **BOYER, Richard**
**Nashville, Tennessee 37212 (US)**

(74) Representative: **Weickmann & Weickmann PartmbB**
**Postfach 860 820**
**81635 München (DE)**

(56) References cited:
**US-A- 6 002 952      US-A1- 2010 016 739**
**US-A1- 2010 191 128      US-A1- 2012 136 242**
**US-A1- 2013 165 802**

• **None**

**Description**

**CROSS-REFERENCE TO RELATED PATENT APPLICATIONS**

This PCT application claims priority to and the benefit of, pursuant to 35 U.S.C. §119(e), U.S. provisional patent application Serial No. 62/049,829, filed September 12, 2014, entitled "METHOD FOR HARMONIC ANALYSIS OF PERIPHERAL VENOUS PRESSURE WAVEFORMS AND APPLICATIONS OF SAME," by Susan S. Eagle, Colleen Brophy, Kyle Mitchell Hocking, Franz Baudenbacher and Richard Boyer.

This PCT application also claims priority to and the benefit of U.S. non-provisional patent application Serial No. 14/853,504, filed September 14, 2015, entitled "HYPOVOLEMIA/HYPERVOLEMIA DETECTION USING PERIPHERAL INTRAVENOUS WAVEFORM ANALYSIS (PIVA) AND APPLICATIONS OF SAME," by Susan S. Eagle, Colleen Brophy, Kyle Mitchell Hocking, Franz Baudenbacher and Richard Boyer.

Some references, which may include patents, patent applications and various publications, are cited and discussed in the description of this invention. The citation and/or discussion of such references is provided merely to clarify the description of the present invention and is not an admission that any such reference is "prior art" to the invention described herein. In terms of notation, hereinafter, "[n]" represents the n-th reference cited in the reference list. For example, [1] represents the first reference cited in the reference list, namely, Wilson, M., D.P. Davis, and R. Coimbra, Diagnosis and monitoring of hemorrhagic shock during the initial resuscitation of multiple trauma patients: a review. The Journal of Emergency Medicine, 2003. 24(4): p. 413-422.

**FIELD OF THE INVENTION**

The present invention relates generally to hypovolemia and/or hypervolemia detection, and more specifically, the present invention relates to systems and methods of detecting hypovolemia and/or hypervolemia using peripheral IV waveform analysis (PIVA) to assess blood volume status of a living subject, and applications of the same.

**BACKGROUND OF THE INVENTION**

Hemorrhagic shock remains the leading preventable cause of death in the casualty care setting [42, 43]. Trauma is the leading cause of death in patients less than 40 years of age and poses a significant economic burden worldwide. Survival is contingent upon early recognition of hemorrhage, appropriate triage, and goal-directed transfusion therapy [43, 44].

Early recognition of hemorrhage and guided fluid administration is critical for providing timely intervention and maintaining end organ viability [1] of human beings and other living animals. For example, Timely damage control surgery (DCS) and restrictive fluid resuscitation (RFR) for wounded soldiers have been shown to significantly improve mortality [45]. While early resuscitation is warranted in patients with hypovolemic shock, over-resuscitation can also have deleterious effects, such as decreased end-organ perfusion, pulmonary edema, and increased mortality [2] [3] [4]. However, there remains a critical technological void for detecting early hemorrhage and resuscitation efficacy, particularly in the clinical setting. Specifically, recognition of subclinical hemorrhage and proper fluid resuscitation has remained elusive, resulting in delayed triage and poor management of the wounded soldier [46]. Often, continuous occult bleeding is not recognized until the onset of hemorrhagic shock and hemodynamic collapse, particularly in a young, healthy soldier with good compensatory mechanisms [47, 48]. Unrecognized hemorrhage leads to delayed triage and DCR, resulting in preventable end-organ damage [49, 50, 35].

Early recognition of hemorrhage is the mainstay of therapy to prevent end-organ damage and to improve survival [34]. Hypovolemic shock and end organ damage are challenging diagnoses in patients with normal heart rate and blood pressure, particularly in patients with good compensatory mechanisms [35]. Specifically, advanced hemorrhagic shock often precedes significant changes in standard vital sign monitoring, particularly in young healthy individuals [5] [1]. In fact, significant vital sign changes do not occur until the patient has lost 15-30% of blood volume, characteristic for Stage II hemorrhage [5]. Even invasive monitoring modalities such as central venous pressure and pulmonary arterial pressure are poor determinants of volume status [6]. Pulmonary artery occlusion pressure and central venous pressure fail to predict ventricular filling volume, cardiac performance, or the response to volume infusion in normal subjects [6]. Furthermore, central monitoring is associated with major vascular complications and central line associated bloodstream infections [7]. More importantly, central venous monitoring offers no survival benefit in critically-ill patients [8] [9].

US 2010/0191128 A1 discloses a system according to the preamble of claim 1.

Therefore, a heretofore unaddressed need exists in the art to address the aforementioned deficiencies and inadequacies.

## SUMMARY OF THE INVENTION

The invention provides a peripheral intravenous (IV) waveform analysis system according to claim 1.

In certain embodiments, the intravascular volume status of the living subject indicates hypovolemia when amplitude decreases greater than a first threshold are detected from the peaks of the peripheral venous pressure frequency spectrum; and the intravascular volume status of the living subject indicates hypervolemia when amplitude increases greater than a second threshold are detected from the peaks of the peripheral venous pressure frequency spectrum. In certain embodiments, the method is performed to the living subject during ultrafiltration/dialysis or diuresis of the living subject. In one embodiment, the method further includes: generating an alert message when the intravascular volume status of the living subject indicates hypovolemia.

In certain embodiments, the system is configured to be used on the living subject during resuscitation of the living subject. In one embodiment, the intravascular volume status of the living subject indicates a return of euvolemia from a hypovolemic state when the living subject is determined to be in the hypovolemic state at an earlier time period, and amplitude increases greater than a third threshold are detected from the peaks of the peripheral venous pressure frequency spectrum. In one embodiment, the intravascular volume status of the living subject indicates over-resuscitation when the living subject is determined to be in an euvolemic state at the earlier time period, and amplitude increases greater than a fourth threshold are detected from the peaks of the peripheral venous pressure frequency spectrum.

In certain embodiments, the system is further adapted to detect efficacy of treatment and the return to euvolemia in the living subject based on the intravascular volume status of the living subject.

In certain embodiments, the peripheral venous signal is acquired by: inserting a peripheral intravenous (IV) catheter into the vein of the living subject; and capturing and recording the peripheral venous signal from the peripheral IV catheter at a sampling rate. In one embodiment, the peripheral IV catheter is a peripherally-inserted central catheter (PICC). In one embodiment, a pressure transducer is directly connected to the peripheral IV catheter, and the peripheral venous signals are captured and recorded by the pressure transducer.

In certain embodiments, the spectral analysis is a spectral fast Fourier transform (FFT) analysis. In certain embodiments, the statistical analysis may include: obtaining a plurality of baseline peaks $\{B_{N-1}\}$ on a baseline peripheral venous pressure frequency spectrum, wherein N is a positive integer, and the plurality of baseline peaks $\{B_{N-1}\}$ respectively corresponds to a plurality of frequencies $\{F_0, F_1, ..., F_N\}$, such that $B_{N-1}$ is a function of $F_{N-1}$ satisfying $B_{N-1} = B_{N-1}(F_{N-1})$, wherein $F_N$ is greater than $F_{N-1}$; obtaining a plurality of peaks $\{P_{N-1}\}$ on the peripheral venous pressure frequency spectrum, wherein the plurality of peaks $\{P_{N-1}\}$ correspond to the plurality of frequencies $\{F_0, F_1, ..., F_N\}$, such that $P_{N-1}$ is a function of $F_{N-1}$ satisfying $P_{N-1} = P_{N-1}(F_{N-1})$; and determining the intravascular volume status of the living subject in real time by comparing the amplitudes of the peaks $\{P_{N-1}\}$ to that of the baseline peaks $\{B_{N-1}\}$ respectively.

In certain embodiments, the baseline peripheral venous pressure frequency spectrum is obtained by: acquiring the peripheral venous signals from the vein of the living subject at an earlier time period; and processing the peripheral venous signals acquired at the earlier time period by the spectral FFT analysis to obtain the baseline peripheral venous pressure frequency spectrum.

In certain embodiments, the system may be capable of detecting at least 6% of blood loss or at least 5.9% of blood volume overload of the living subject.

In certain embodiments, the plurality of peaks $\{P_{N-1}\}$ includes a first peak $P_0$ corresponding to a first frequency $F_0$ and a second peak $P_1$ corresponding to a second frequency $F_1$. In certain embodiments, the first peak $P_0$ corresponding to the first frequency $F_0$ is associated with a respiratory rate of the living subject; and the second peak $P_1$ corresponding to the second frequency $F_1$ is associated with a heart rate of the living subject.

In certain embodiments, the system is for determining hypovolemia, hypervolemia and vascular tone of a living subject based on an intravascular volume status of the living subject. In certain embodiments, the system is configured to acquire, continuously for a time period from $T_0$ to $T_2$, peripheral venous signals from a vein of the living subject, wherein the time period is divided into a first time period from $T_0$ to $T_1$, and a second time period from $T_1$ to $T_2$; processing the peripheral venous signals acquired at the first time period to obtain a baseline peripheral venous pressure frequency spectrum; obtaining a plurality of baseline peaks $\{B_{N-1}\}$ on the baseline peripheral venous pressure frequency spectrum, wherein N is a positive integer, and the plurality of baseline peaks $\{B_{N-1}\}$ respectively corresponds to a plurality of frequencies $\{F_0, F_1, ..., F_N\}$, such that $B_{N-1}$ is a function of $F_{N-1}$ satisfying $B_{N-1} = B_{N-1}(F_{N-1})$, wherein $F_N$ is greater than $F_{N-1}$; processing the peripheral venous signals acquired at the second time period to obtain a peripheral venous pressure frequency spectrum; obtaining a plurality of peaks $\{P_{N-1}\}$ on the peripheral venous pressure frequency spectrum, wherein the plurality of peaks $\{P_{N-1}\}$ correspond to the plurality of frequencies $\{F_0, F_1, ..., F_N\}$, such that $P_{N-1}$ is a function of $F_{N-1}$ satisfying $P_{N-1} = P_{N-1}(F_{N-1})$; and determining the intravascular volume status of the living subject at the second time period by comparing amplitudes of the peaks $\{P_{N-1}\}$ to that of the baseline peaks $\{B_{N-1}\}$ respectively. The intravascular volume status of the living subject at the second time period indicates hypovolemia or hypervolemia when amplitude changes greater than a threshold are detected from the baseline peaks $\{B_{N-1}\}$ to the peaks $\{P_{N-1}\}$.

In certain embodiments, the intravascular volume status of the living subject at the second time period indicates hypovolemia when amplitude decreases are detected greater than a first threshold from the baseline peaks $\{B_{N-1}\}$ to the peaks $\{P_{N-1}\}$. In certain embodiments, the intravascular volume status of the living subject at the second time period indicates hypervolemia when amplitude increases greater than a second threshold are detected from the baseline peaks $\{B_{N-1}\}$ to the peaks $\{P_{N-1}\}$.

In certain embodiments, the system is configured to be used on the living subject during resuscitation of the living subject. In one embodiment, the intravascular volume status of the living subject at the second time period indicates a return of euvolemia from a hypovolemic state when the living subject is determined to be in the hypovolemic state at the first time period, and amplitude increases greater than a third threshold are detected from the baseline peaks $\{B_{N-1}\}$ to the peaks $\{P_{N-1}\}$. In one embodiment, the intravascular volume status of the living subject at the second time period indicates over-resuscitation when the living subject is determined to be in an euvolemic state at the first time period, and amplitude increases greater than a fourth threshold are detected from the baseline peaks $\{B_{N-1}\}$ to the peaks $\{P_{N-1}\}$.

In certain embodiments, the peripheral venous signals are acquired by: inducing anesthesia on the living subject; inserting a peripheral intravenous (IV) catheter into the vein of the living subject, wherein the vein is an upper extremity vein; and capturing and recording the peripheral venous signals from the peripheral IV catheter at a sampling rate of about 1 kHz. In one embodiment, the peripheral IV catheter is a peripherally-inserted central catheter (PICC). In certain embodiments, a pressure transducer is used to be directly connected to the peripheral IV catheter, and the peripheral venous signals are captured and recorded by the pressure transducer.

In certain embodiments, the peripheral venous signals are processed by a spectral fast Fourier transform (FFT) analysis to obtain the baseline peripheral venous pressure frequency spectrum and the peripheral venous pressure frequency spectrum, respectively.

In certain embodiments, the plurality of peaks $\{P_{N-1}\}$ includes a first peak corresponding to a first frequency $F_0$ and a second peak corresponding to a second frequency $F_1$. In one embodiment, the first peak corresponding to the first frequency $F_0$ is associated with a respiratory rate of the living subject; and the second peak corresponding to the second frequency $F_1$ is associated with a heart rate of the living subject.

In certain embodiments, the intravascular volume status of the living subject indicates hypovolemia when amplitude decreases greater than a first threshold are detected from the peaks of the peripheral venous pressure frequency spectrum; and the intravascular volume status of the living subject indicates hypervolemia when amplitude increases greater than a second threshold are detected from the peaks of the peripheral venous pressure frequency spectrum. In certain embodiments, the system is applied to the living subject during ultrafiltration/dialysis or diuresis of the living subject. In one embodiment, the processing device is further configured to: generate an alert message when the intravascular volume status of the living subject indicates hypovolemia.

In certain embodiments, the system is applied to the living subject during resuscitation of the living subject. In one embodiment, the intravascular volume status of the living subject indicates a return of euvolemia from a hypovolemic state when the living subject is determined to be in the hypovolemic state at an earlier time period, and amplitude increases greater than a third threshold are detected from the peaks of the peripheral venous pressure frequency spectrum. In one embodiment, the intravascular volume status of the living subject indicates over-resuscitation when the living subject is determined to be in an euvolemic state at the earlier time period, and amplitude increases greater than a fourth threshold are detected from the peaks of the peripheral venous pressure frequency spectrum.

In certain embodiments, the processing device is further configured to: detect efficacy of treatment and the return to euvolemia in the living subject based on the intravascular volume status of the living subject.

In certain embodiments, the processing device is communicatively connected to the peripheral IV device through a wireless connection.

In certain embodiments, the peripheral IV device includes: a peripheral IV catheter being inserted into the vein of the living subject; and a monitoring device connected to the peripheral IV catheter, configured to capture and record the peripheral venous signals from the peripheral IV catheter at a sampling rate. In one embodiment, the peripheral IV catheter is a peripherally-inserted central catheter (PICC). In certain embodiments, the monitoring device comprises a pressure transducer directly connected to the peripheral IV catheter, wherein the peripheral venous signals are captured and recorded by the pressure transducer.

In certain embodiments, the processing device is a computing device, which may be a desktop computer, a laptop computer, a smartphone, a tablet device, or any other computing devices with processors to perform the processing functions.

In certain embodiments, the spectral analysis is a spectral fast Fourier transform (FFT) analysis. In certain embodiments, the statistical analysis may include: obtaining a plurality of baseline peaks $\{B_{N-1}\}$ on a baseline peripheral venous pressure frequency spectrum, wherein N is a positive integer, and the plurality of baseline peaks $\{B_{N-1}\}$ respectively corresponds to a plurality of frequencies $\{F_0, F_1, ..., F_N\}$, such that $B_{N-1}$ is a function of $F_{N-1}$ satisfying $B_{N-1} = B_{N-1}(F_{N-1})$, wherein $F_N$ is greater than $F_{N-1}$; obtaining a plurality of peaks $\{P_{N-1}\}$ on the peripheral venous pressure frequency spectrum, wherein the plurality of peaks $\{P_{N-1}\}$ correspond to the plurality of frequencies $\{F_0, F_1, ..., F_N\}$, such that $P_{N-1}$ is a function of $F_{N-1}$ satisfying $P_{N-1} = P_{N-1}(F_{N-1})$; and determining the intravascular volume status of the living subject in real time by comparing the amplitudes of the peaks $\{P_{N-1}\}$ to that of the baseline peaks $\{B_{N-1}\}$ respectively.

In certain embodiments, the baseline peripheral venous pressure frequency spectrum is obtained by: acquiring the peripheral venous signals from the vein of the living subject at an earlier time period; and processing the peripheral venous signals acquired at the earlier time period by the spectral FFT analysis to obtain the baseline peripheral venous pressure frequency spectrum.

In certain embodiments, the plurality of peaks $\{P_{N-1}\}$ includes a first peak $P_0$ corresponding to a first frequency $F_0$ and a second peak $P_1$ corresponding to a second frequency $F_1$. In certain embodiments, the first peak $P_0$ corresponding to the first frequency $F_0$ is associated with a respiratory rate of the living subject; and the second peak $P_1$ corresponding to the second frequency $F_1$ is associated with a heart rate of the living subject.

In certain embodiments, the PIVA system further includes: a pump connected to the living subject to perform liquid exchange to the living subject; and a pump controlling mechanism communicatively connected to the processing device, configured to control the pump by intermittently pausing the pump or subtract the pump signal when the peripheral IV device acquires the peripheral venous signals, and restarting the pump when the peripheral IV device does not acquire the peripheral venous signals. The processing device is further configured to send a signal to the pump controlling mechanism to notify the pump controlling mechanism to control the pump. In certain embodiments, the pump may be a dialysis pump, a cardiopulmonary bypass pump, an extracorporeal membrane oxygenation (ECMO), or an infusion pump.

In certain embodiments, detection of hypovolemia through the system as described above may be performed through the analysis of amplitude increases in the peaks with higher frequencies, such as $F_2$, as well as the amplitude changes in $F_1$.

These and other aspects of the present invention will become apparent from the following description of the preferred embodiments taken in conjunction with the following drawings, although variations and modifications thereof may be affected without departing from the scope of the novel concepts of the disclosure.

## BRIEF DESCRIPTION OF THE DRAWINGS

The accompanying drawings illustrate one or more embodiments of the invention and, together with the written description, serve to explain the principles of the invention. Wherever possible, the same reference numbers are used throughout the drawings to refer to the same or like elements of an embodiment.

FIG. 1 shows a PIVA system according to certain embodiments of the present invention.

FIG. 2 shows a flowchart of a method for detecting hypovolemia/hypervolemia of a living subject according to certain embodiments of the present invention.

FIG. 3 shows charts of the peripheral venous waveforms and the Fourier transformation of the signals in states of hypovolemia (A), euvolemia (B), and hypervolemia (C) in the porcine model, according to certain embodiments of the present invention.

FIG. 4A shows a chart of the $F_1$ amplitude of the hemorrhage and transfusion of blood in the porcine model for volume status according to certain embodiments of the present invention.

FIG. 4B shows a chart of mean arterial pressure (MAP), heart rate (HR), and shock index (SI) to the blood volume of the hemorrhage and transfusion of blood in the porcine model for volume status according to certain embodiments of the present invention.

FIG. 5A shows a chart of the $F_1$ amplitude of fluid administration to mild hypervolemia in the porcine model for volume status according to certain embodiments of the present invention.

FIG. 5B shows a chart of MAP, HR, and SI to the blood volume of fluid administration to mild hypervolemia in the porcine model for volume status according to certain embodiments of the present invention.

FIG. 6 shows the receiver operator curves (ROC) for detection of (A) hypovolemia (>200mL hemorrhage, 5.9%) and (B) hypervolemia (>200mL fluid administration, 5.9%) for the peripheral venous signal, HR, MAP, and SI according to certain embodiments of the present invention.

FIG. 7 shows a table of patient demographics in a controlled human hemorrhagic model according to certain embodiments of the present invention.

FIG. 8 shows charts of the peripheral venous waveform and the Fourier transformation of the signals at baseline and after autologous blood removal in the controlled human hemorrhagic model according to certain embodiments of the present invention.

FIG. 9 shows charts of hemodynamic measurements for (A) the $F_1$ amplitude, (B) HR, (C) diastolic pulmonary artery pressure (dPAP) and (D) MAP in the controlled human hemorrhagic model at baseline and following hemorrhage at 250 mL and 500mL according to certain embodiments of the present invention.

FIG. 10A shows the ROC curves for detection of the $F_1$ amplitude, dPAP, HR and SI according to certain embodiments of the present invention.

FIG. 10B shows a table of the area under the curve (AUC), standard error (SE) and 95% confidence interval for the data as shown in FIG. 8A according to certain embodiments of the present invention.

FIG. 11A shows the $F_1$ amplitudes with (+PPV) and without (-PPV) positive pressure ventilation according to certain embodiments of the present invention.

FIG. 11B shows the $F_0$ amplitudes with (+PPV) and without (-PPV) positive pressure ventilation according to certain embodiments of the present invention.

FIG. 12 shows (A) PIVA signal and (B) shock index for detecting hemorrhage in a porcine animal model (n=8), according to certain embodiments of the present invention.

FIG. 13 shows (A) PIVA signal and (B) shock index for detecting hemorrhage in a porcine animal model (n=8), according to certain embodiments of the present invention.

## DETAILED DESCRIPTION OF THE INVENTION

The invention will now be described more fully hereinafter with reference to the accompanying drawings, in which exemplary embodiments of the invention are shown. This invention may, however, be embodied in many different forms and should not be construed as limited to the embodiments set forth herein. Rather, these embodiments are provided so that this disclosure will be thorough and complete, and will fully convey the scope of the invention to those skilled in the art. Like reference numerals refer to like elements throughout.

The terms used in this specification generally have their ordinary meanings in the art, within the context of the invention, and in the specific context where each term is used. Certain terms that are used to describe the invention are discussed below, or elsewhere in the specification, to provide additional guidance to the practitioner regarding the description of the invention. For convenience, certain terms may be highlighted, for example using italics and/or quotation marks. The use of highlighting has no influence on the scope and meaning of a term; the scope and meaning of a term are the same, in the same context, whether or not it is highlighted. It will be appreciated that the same thing can be said in more than one way. Consequently, alternative language and synonyms may be used for any one or more of the terms discussed herein, nor is any special significance to be placed upon whether or not a term is elaborated or discussed herein. Synonyms for certain terms are provided. A recital of one or more synonyms does not exclude the use of other synonyms. The use of examples anywhere in this specification including examples of any terms discussed herein is illustrative only, and in no way limits the scope and meaning of the invention or of any exemplified term. Likewise, the invention is not limited to various embodiments given in this specification.

It will be understood that when an element is referred to as being "on" another element, it can be directly on the other element or intervening elements may be present there between. In contrast, when an element is referred to as being "directly on" another element, there are no intervening elements present. As used herein, the term "and/or" includes any and all combinations of one or more of the associated listed items.

It will be understood that, although the terms first, second, third, etc. may be used herein to describe various elements, components, regions, layers and/or sections, these elements, components, regions, layers and/or sections should not be limited by these terms. These terms are only used to distinguish one element, component, region, layer or section from another element, component, region, layer or section. Thus, a first element, component, region, layer or section discussed below could be termed a second element, component, region, layer or section without departing from the teachings of the invention.

The terminology used herein is for the purpose of describing particular embodiments only and is not intended to be limiting of the invention. As used herein, the singular forms "a", "an" and "the" are intended to include the plural forms as well, unless the context clearly indicates otherwise. It will be further understood that the terms "comprises" and/or "comprising", or "includes" and/or "including" or "has" and/or "having" when used in this specification specify the presence of stated features, regions, integers, steps, operations, elements, and/or components, but do not preclude the presence or addition of one or more other features, regions, integers, steps, operations, elements, components, and/or groups thereof.

Furthermore, relative terms, such as "lower" or "bottom" and "upper" or "top", may be used herein to describe one element's relationship to another element as illustrated in the Figures. It will be understood that relative terms are intended to encompass different orientations of the device in addition to the orientation depicted in the Figures. For example, if the device in one of the figures is turned over, elements described as being on the "lower" side of other elements would then be oriented on "upper" sides of the other elements. The exemplary term "lower" can, therefore, encompass both an orientation of "lower" and "upper", depending on the particular orientation of the figure. Similarly, if the device in one of the figures is turned over, elements described as "below" or "beneath" other elements would then be oriented "above" the other elements. The exemplary terms "below" or "beneath" can, therefore, encompass both an orientation of above and below.

Unless otherwise defined, all terms (including technical and scientific terms) used herein have the same meaning as commonly understood by one of ordinary skill in the art to which this invention belongs. It will be further understood that terms, such as those defined in commonly used dictionaries, should be interpreted as having a meaning that is consistent with their meaning in the context of the relevant art and the present disclosure, and will not be interpreted in an idealized or overly formal sense unless expressly so defined herein.

As used herein, "around", "about", "substantially" or "approximately" shall generally mean within 20 percent, preferably within 10 percent, and more preferably within 5 percent of a given value or range. Numerical quantities given herein are approximate, meaning that the term "around", "about", "substantially" or "approximately" can be inferred if not expressly stated.

As used herein, the terms "comprise" or "comprising", "include" or "including", "carry" or "carrying", "has/have" or "having", "contain" or "containing", "involve" or "involving" and the like are to be understood to be open-ended, *i.e.*, to mean including but not limited to.

As used herein, the term "hemodynamic" generally refers to blood movement, and "hemodynamic resuscitation" generally refers to increasing blood movement (or blood pressure) in a patient experiencing symptoms of compensated shock (e.g., based on a "hemodynamic score" or "resuscitation score").

As used herein, the term "peripheral intravenous waveform analysis" or its abbreviation "PIVA" refers to an analysis of the peripheral venous waveforms measured from a vein of a living subject through a standard peripheral intravenous (IV) catheter.

As used herein, the term "hypovolemia" refers to a medical condition of decreased blood volume, and more specifically a decrease in volume of blood plasma. In certain embodiments, hypovolemia stems from loss of blood volume due to hemorrhage, dehydration or intravascular water loss.

As used herein, the term "hypervolemia" refers to a medical condition of fluid overload (i.e., having too much fluid) in the blood. In certain embodiments, hypervolemia stems from compromised regulatory mechanisms for sodium handling, such as congestive heart failure (CHF) or renal failure, or due to iatrogenic fluid administration.

## OVERVIEW OF THE INVENTION

The ideal hemodynamic monitoring system for guiding fluid therapy would predict patient volume status accurately with minimal risks [36]. Unfortunately, existing dynamic systems and methods for assessing volume status, such as pulse pressure variation (PPV), stroke volume variation (SVV), and plethysmographic wave respiratory variation, may predict fluid responsiveness, but do not directly measure volume status, and they have not been proven to detect iatrogenic volume overload during resuscitation [37, 38]. These existing non-invasive dynamic monitoring systems, such as SVV and PPV, rely on intrathoracic effects on left ventricular stroke volume during mechanical ventilation [10, 11]. Furthermore, these techniques depend on heart-lung interactions during mechanical ventilation for detection of hypovolemia. [37, 51] This critical limitation renders the techniques ineffective in the spontaneously breathing patient [52].

In an effort to address the pitfalls associated with the existing central venous monitoring modalities, peripheral venous pressure (PVP) monitoring has been increasingly explored as an alternative for determining intravascular volume status. There is a good correlation between trends in absolute PVP and central venous pressure (CVP) measurements in critically ill patients [13] [14]. However, even trends of CVP, and therefore PVP, are not reliable indicators of volume status, resulting in a need to modify this approach. One such method, cuff-occlusion rate of rise of PVP (CORRP), has been shown to correlate with volume status in critically-ill patients, but is limited by operator-dependent, non-continuous measurements to assess volume status [15]. Another method used peripheral venous waveform spectral analysis for continuous beat-to-beat monitoring of intravascular volume status to quantify changes associated with lower body negative pressure, a simulation of hemorrhage, in spontaneously breathing subjects [16]. However, lower body negative pressure results in vasodilation and may not truly represent the physiological responses to hemorrhage. In other words, there is no existing non-invasive method of accurately assessing patient blood volume status in intrathoracic pressure changes without ventilation-induced or negative pressure. The current gold-standard measurement requires either echocardiography or a pulmonary artery catheter, and both of these measurements have wide variability in their accuracy.

Recently, *Sileshi et al.* reported that peripheral intravenous waveform analysis (PIVA) can detect early Stage 1 hemorrhage, during perioperative autologous blood donation in patients undergoing cardiac surgery [17]. Moreover, this study demonstrated that PIVA was more sensitive to acute changes in circulating blood volume than standard vital signs and invasive pulmonary arterial monitoring.

Moreover, since not all wounded soldiers require endotracheal intubation and positive pressure ventilation, there is an acute need for accurate and sensitive volume status monitoring in wounded, spontaneously breathing soldiers. There is a need for a point of care monitor that detect small direct changes in volume status and warns of impending hemodynamic collapse in the combat casualty care setting.

Accordingly, aspects of the present invention relates to systems of detecting early stage hemorrhage using PIVA to assess blood volume status of a living subject, which may include human beings and/or other animals, and applications of the same.

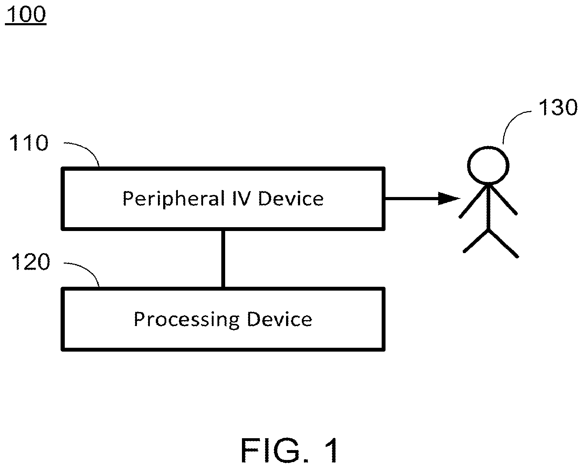

FIG. 1 shows a PIVA system according to certain embodiments of the present invention. As shown in FIG. 1, the PIVA system 100 includes: a peripheral IV device 110 and a processing device 120. The processing device 120 is communicatively connected to the peripheral IV device 110. In certain embodiments, the connection between the peripheral IV device 110 and the processing device 120 may be through a network, which may be implemented by a wired connection or a wireless connection. Examples of the network may include without being limited to, a local area network (LAN), a wide area network (WAN), the Internet, or any other types of network.

The peripheral IV device 110 is configured to acquire, from a vein of a living subject 130, peripheral venous signals. In certain embodiments, the living subject may be a human being, or may be other animals. In one embodiment, the living subject may be a human patient, and the patient may be awake and spontaneously breathing/moving, or may be induced with anesthesia such that the patient is not spontaneously moving. In certain embodiments, the peripheral IV device 110 may include a peripheral IV catheter being inserted into the vein of the living subject 130, and a monitoring device connected to the peripheral IV catheter. The monitoring device is configured to capture and record the peripheral venous signals from the peripheral IV catheter at a sampling rate. In certain embodiments, the monitoring device may include a pressure transducer directly connected to the peripheral IV catheter, such that the peripheral venous signals are captured and recorded by the pressure transducer.

The processing device 120 is configured to: receive the peripheral venous signals from the peripheral IV monitoring device; perform a spectral process and analysis on the peripheral venous signal to obtain a peripheral venous pressure frequency spectrum; and perform a statistical analysis on amplitudes of peaks of the peripheral venous pressure frequency spectrum to determine the blood volume status of the living subject in real time. In certain embodiments, the processing device 120 may be a computing device, which may be a desktop computer, a laptop computer, a smartphone, a tablet device, or any other computing devices with processors to perform the processing functions. In certain embodiments, the spectral analysis may be a spectral fast Fourier transform (FFT) analysis.

In certain embodiments, the PIVA system 100 further includes: a pump (not shown) connected to the living subject to perform liquid exchange to the living subject; and a pump controlling mechanism (not shown) communicatively connected to the processing device, configured to control the pump by intermittently pausing the pump or subtract the pump signal when the peripheral IV device acquires the peripheral venous signals, and restarting the pump when the peripheral IV device does not acquire the peripheral venous signals. In certain embodiments, the pump may be a dialysis pump, a cardiopulmonary bypass pump, an extracorporeal membrane oxygenation (ECMO), or an infusion pump. In this case, the processing device 120 is further configured to send a signal to the pump controlling mechanism to notify the pump controlling mechanism to control the pump.

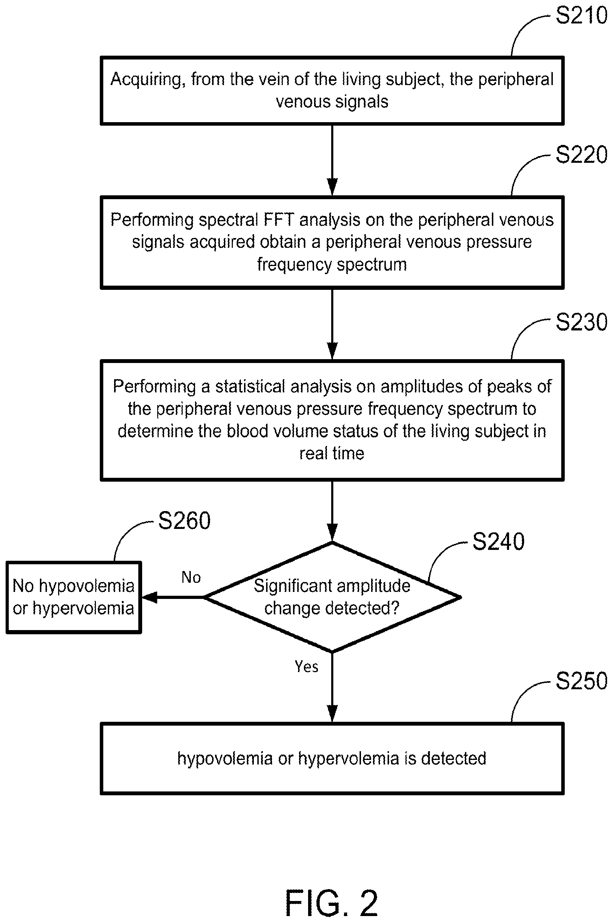

FIG. 2 shows a flowchart for detecting hypovolemia/hypervolemia of a living subject according to certain embodiments of the present invention. As shown in FIG. 2, at step S210, the peripheral IV device 110 acquires the peripheral venous signals from the vein of the living subject. At step S220, upon receiving the peripheral venous signals from the peripheral IV device 110, the processing device 120 performs a spectral process and analysis, such as the spectral FFT analysis, on the peripheral venous signal to obtain a peripheral venous pressure frequency spectrum. At step S230, the processing device 120 performs a statistical analysis on amplitudes of peaks of the peripheral venous pressure frequency spectrum to determine the blood volume status of the living subject in real time. At step S240, the processing device 120 determines whether a significant amplitude change of the peaks is detected. If so, at step S250, the processing device 120 determines that the living subject has hypovolemia or hypervolemia, depending on the amplitude change. If not, at step S260, the processing device 120 determines that the living subject has no hypovolemia or hypervolemia.

Specifically, the steps S210 and S220 may be performed continuously, such that at two different time period, two sets of the peripheral venous pressure frequency spectrums may be obtained. For example, for a time period from $T_0$ to $T_2$, the time period may be divided into a first time period from $T_0$ to $T_1$, and a second time period from $T_1$ to $T_2$., and each of the first time period and the second time period may be used to obtain a separate set of peripheral venous pressure frequency spectrums. In certain embodiments, the time period may be divided into more than two time periods, and multiple sets of peripheral venous pressure frequency spectrums may be obtained. In certain embodiments, the peripheral venous pressure frequency spectrum obtained at an earlier time may be used as a baseline peripheral venous pressure frequency spectrum. Thus, the statistical analysis at step S230 may be performed by obtaining a plurality of baseline peaks $\{B_{N-1}\}$ from a lower frequency side on a baseline peripheral venous pressure frequency spectrum, where N is a positive integer, and the plurality of baseline peaks $\{B_{N-1}\}$ respectively corresponds to a plurality of frequencies $\{F_0, F_1, ..., F_N\}$, such that $B_{N-1}$ is a function of $F_{N-1}$ satisfying $B_{N-1} = B_{N-1}(F_{N-1})$, wherein $F_N$ is greater than $F_{N-1}$. In other words, the baseline peaks may include a first baseline peak $B_0$ corresponding to a first frequency $F_0$, a second baseline peak $B_1$ corresponding to a second frequency $F_1$, a third baseline peak $B_2$ corresponding to a third frequency $F_2$ ..., and the second frequency $F_1$ is greater than the first frequency $F_0$. Then, a plurality of peaks $\{P_{N-1}\}$ may be obtained on the peripheral venous pressure frequency spectrum currently obtained, where the plurality of peaks $\{P_{N-1}\}$ correspond to the plurality of frequencies $\{F_0, F_1, ..., F_N\}$, such that $P_{N-1}$ is a function of $F_{N-1}$ satisfying $P_{N-1} = P_{N-1}(F_{N-1})$. For example, the peaks may include a first peak $P_0$ corresponding to the first frequency $F_0$, a second peak $P_1$ corresponding to the second frequency $F_1$, a third peak $P_2$ corresponding to the third frequency $F_2$ .... In certain embodiments, the number of peaks on the peripheral venous pressure frequency spectrum equals to the number of baseline peaks on the baseline peripheral venous pressure frequency spectrum. In this way, the intravascular volume status of the living subject may be determined in real time by comparing the amplitudes of the peaks to that of the corresponding baseline peaks, respectively.

In certain embodiments, the intravascular volume status of the living subject at the second time period indicates hypovolemia when amplitude decreases are detected greater than a first threshold from the baseline peaks $\{B_{N-1}\}$ to the peaks $\{P_{N-1}\}$. In certain embodiments, the intravascular volume status of the living subject at the second time period indicates hypervolemia when amplitude increases greater than a second threshold are detected from the baseline peaks $\{B_{N-1}\}$ to the peaks $\{P_{N-1}\}$. For example, if there is a significant decrease in the $F_1$ amplitude of the second peak of the peripheral venous pressure frequency spectrum that reaches the first threshold, the processing device 120 will determine that hypovolemia (e.g., hemorrhage or intravascular water loss, etc.) has occurred in the living subject. On the other hand, if there is a significant increase in the $F_1$ amplitude of the second peak of the peripheral venous pressure frequency spectrum that reaches the second threshold, the processing device 120 will determine that hypervolemia (e.g., congestive heart failure, renal failure, iatrogenic fluid administration, etc.) has occurred in the living subject. It should be noted that, in addition to the $F_1$ amplitude of the second peak $P_1$, the amplitude of other peaks, such as the first peak $P_0$, the third peak $P_2$, and any subsequent peaks, may also be used to determine hypovolemia or hypervolemia of the living subject.

In certain embodiments, the method is performed to the living subject during resuscitation of the living subject. In one embodiment, the intravascular volume status of the living subject at the second time period indicates a return of euvolemia from a hypovolemic state when the living subject is determined to be in the hypovolemic state at the first time period, and amplitude increases greater than a third threshold are detected from the baseline peaks $\{B_{N-1}\}$ to the peaks $\{P_{N-1}\}$. In one embodiment, the intravascular volume status of the living subject at the second time period indicates over-resuscitation when the living subject is determined to be in an euvolemic state at the first time period, and amplitude increases greater than a fourth threshold are detected from the baseline peaks $\{B_{N-1}\}$ to the peaks $\{P_{N-1}\}$.

In certain embodiments, the actual volume calculation may be performed based on a large logic table, with interpolation, that will be generated based on a population study and can be further fit to the specific patient in a chronic-use device. An example of the logic table is provided as follows.

Example Logic Table

| H1- amplitude | H2- amplitude | H3- amplitude | Volume Status Ratio |
|---|---|---|---|
| 0.05 | 0.023 | 0.018 | 1.1 |
| 0.071 | 0.035 | 0.025 | 1.1 |
| 0.119 | 0.652 | 0.017 | 0.8 |

Further, in certain embodiments, a regression model may be provided to calculate the volume status ratio. For example:

$$\text{Volume Status Ratio} = b_0 + b_1 \cdot p_1 + b_2 \cdot p_2 + \ldots + b_N \cdot p_N$$

where $b_0$, $b_1$, $b_2$, ..., $b_N$ are linear regression coefficients determined in a multivariate regression model for each independent parameter $p_i$. In certain embodiments, the parameters may include:

1. Amplitudes (or powers) of each harmonic in the frequency domain
2. Noise floor amplitude
3. HR
4. Time domain amplitude of peripheral venous pressure

In certain embodiments, the method may be capable of detecting at least 6% of blood loss of the living subject.

In certain embodiments, the system 100 may be implemented by an instrumented IV catheter interfaced with a smart phone device for early hemorrhage detection and guided fluid therapy to improve survival in the casualty care setting. The device uses: (1) A ruggedized miniature pressure transducer integrated into an IV catheter, (2) real-time algorithm for early hemorrhage detection and guided goal-directed fluid resuscitation (3) wireless immediate data transfer for remote or centralized monitoring and intervention. In certain embodiments, the device may involve a portable, lightweight (<50 grams) sensor that attaches directly to a standard peripheral IV catheter interfaced to a Bluetooth radio for transmission to a Smartphone device. The form factor of the device will be comparable to a USB stick. The cost of the device may be USD <$25 making it feasible to integrate the device into every IV catheter used in casualty care and trauma settings. Further, the device may utilize immediate data transfer and storage to a secure database and mobile application for remote Damage Control Resuscitation and emergency telemedicine. In addition to immediate graphic display for field medics, the smart IV will have Bluetooth technology allowing data transfer to a Smart phone mobile application. Bidirectional data transfer and feedback from remote clinical practitioners provides emergency telemedicine assistance to field medics in the battlefield. Further, physiologically data can be uploaded to a server, providing an electronic medical record for all care providers throughout the resuscitation period. Such device may be potentially relevant in military use, as the military needs a robust point-of-care method for early hemorrhage detection in the wounded soldier. This device will be rugged, wireless, lightweight and portable with minimal, self-contained energy requirements, conducive to austere environments.

As described above, the PIVA system 100 includes the processing device 120, which serves as a controller device that is configured to receive the peripheral venous signals from the monitoring device of the peripheral IV device 110. The processing device 120 may then perform the Fourier transform on the peripheral venous signals of the time domain data to process the signal to obtain a spectrum. Once the fourier spectrum is generated, the second peak at the frequency $F_1$ that correspond to the heart rate or another hemodynamic parameter's amplitude or power is measured and each resonant frequency of that hemodynamic parameter's amplitude or power is also measured. The amplitudes and/or powers are input into the algorithm that weights each resonant frequency and outputs a measurement of a hemodynamic parameter. Additional inputs may also be required, including but not limited to the following: age, weight, gender, and height, these variables may be input into the algorithm to determine a more accurate depiction of the patient's hemodynamic state. Based on the Fourier transform of the piezoelectric signal the processor is used to generate a resuscitation score based on the hemodynamic parameter.

The invention relates to a system adapted to perform methods for peripheral venous pressure analysis algorithm that uses spectral analysis to estimate intravascular volume status, and its applications. In certain aspects, the invention recites, among other things:

1) Harmonic peripheral venous pressure waveform analysis algorithm.
2) Measuring peripheral venous pressure frequency spectra for determination of real-time volume status.
3) A venous pressure monitor algorithm that can distinguish between euvolemia and hypervolemia (all current technologies stop at euvolemia).
4) Assessing volume status in a spontaneously breathing patient.
5) A volume status monitor that uses a peripheral IV.
6) A closed loop system for controlling volume status with a peripheral venous pressure monitor and intravenous fluid pump.

In the following examples, the inventors have utilized PIVA in different models, including a porcine hemorrhage-resuscitation model and a controlled human hemorrhagic model, to analyze and study dynamic volume changes and shifts in the peripheral venous waveforms. The tests in the examples are performed in standardized settings in order to test the hypothesis that PIVA is more sensitive and specific than standard and invasive monitors for detecting and quantitating acute hemorrhage, as well as resuscitation to euvolemia and iatrogenic fluid overload. Further, the accuracy and linearity of the PIVA estimation of blood volume has been evaluated as compared to absolute volume removal and administration.

**EXAMPLE 1**

**TEST IN PORCINE HEMORRHAGE-RESUSCITATION MODEL**

In this example, a test has been performed using PIVA in a porcine hemorrhage-resuscitation model to study dynamic volume shifts in a standardized setting. Under an approved Institutional Animal Care and Use Committee protocol, 8 adult Yorkshire pigs, each weighing 45+/-0.8 kg, were monitored non-invasively with a noninvasive blood pressure cuff, 5-lead electrocardiogram, and pulse oximeter (SurgiVet, Norwell, Boston, MA). Each animal was induced with general anesthesia Telazol 2 mg, Ketamine 50 mg, and Xylazine 2 mg given via an ear vein. After intubation with a cuffed 5.0 ID endotracheal tube the pigs were ventilated with a volume controlled ventilator (Hallowell EMC, MA, USA) with a volume-controlled mode of 8mL/kg tidal volume with a positive end-expiratory pressure of 5 cm $H_2O$, I:E ratio 1:2, and a $FiO_2$ 1.0. Respiratory rate (16-22 breaths/minute) was titrated to maintain an end-tidal $CO_2$ of 35-40 mmHg.

Anesthesia was maintained with 1% isoflurane (Primal Healthcare, Boston, MA). Surgical exposure of the femoral artery and vein was obtained. A 6Fr catheter (Mila International, Erlanger, KY) was inserted directly into the femoral vein and sutured in place. A 20g angiocatheter (Mila International, Erlanger, KY) was placed in the femoral artery for continuous blood pressure measurements. A 20g peripheral IV (Smiths Medical, Dublin, OH) was inserted into the front extremity of each animal. Details of the test are provided as follows:

Peripheral Venous Waveform Acquisition

After induction of anesthesia, a 20 gauge peripheral intravenous (IV) catheter was inserted into an upper extremity vein and directly connected to a pressure transducer (ADInstruments, Colorado Springs, CO) via standard high- pressure tubing. The IV catheter was dedicated to obtaining venous waveforms and was not used for infusion of fluids or drug delivery during the test. Peripheral venous waveform tracings were recorded continuously throughout the procedure and analyzed on LabChart (ADInstruments, Colorado Springs, CO). Peripheral intravenous waveform data was captured at 1 kHz to allow adequate sampling to perform spectral analysis of the waveform data.

Hemorrhage Protocol

After induction of anesthesia, baseline measurements of the peripheral venous signal were obtained. Standard IV tubing was attached proximally to the femoral venous line and distally to a sterile collection bag, producing a closed system. Intravenous Heparin 10 units/kg was administered immediately prior to hemorrhage. Venous blood was removed via gravity in a stepwise fashion of 50 mL per minute over a 10-minute period. Vital signs including heart rate, arterial blood pressure, and peripheral venous waveforms were continuously recorded throughout the procedure. The procedure was terminated at 400 mL blood removal or mean arterial pressure (MAP) < 40mmHg.

Volume Resuscitation

Immediately following the hemorrhage protocol, the entire autologous blood was returned to the pig at a continuous rate of 50 mL/min over 10 minutes through the femoral venous catheter. Vital signs were continuously recorded throughout this process. Following return of the autologous blood, each pig was considered to be in an euvolemic state.

Iatrogenic Volume Overload

Following volume resuscitation to a euvolemic state, warm (40 °C) 500 mL Plasmalyte (Baxter International, Deerfield, IL) balanced crystalloid solution was administered through the femoral venous catheter over a 10-minute period. Continuous hemodynamic monitoring was performed as previously described.

Spectral Analysis

The spectral fast Fourier transform (FFT) analysis of peripheral venous pressure was performed using an 8K sampling window with no window overlap. Data was recorded at a sampling rate of 1 kHz necessitating 8 seconds of continuous time-domain signal to perform the 8K-FFT spectral analysis. Following Fourier transformation, the amplitude of each frequency peak was calculated in LabChart. The peak amplitude associated with the heart rate was used to acquire $F_1$ (the fundamental frequency) amplitude of the signal. Data was captured in triplicate for each point used in analysis.

Results

All 8 pigs successfully underwent hemorrhage, resuscitation, and volume overload without complications. Conversion of the time domain signal into the frequency domain was performed to determine the amplitude of each of the fundamental frequency recorded during the experiment.

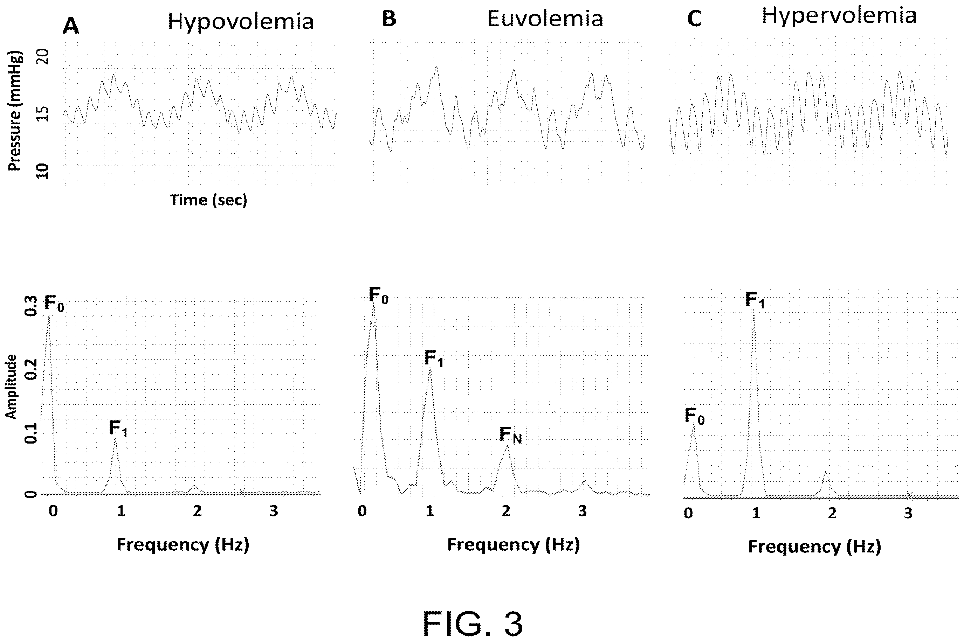

FIG. 3 shows charts of the peripheral venous waveforms and the Fourier transformation of the signals in states of hypovolemia (A), euvolemia (B), and hypervolemia (C) in the porcine model, according to certain embodiments of the present invention. As shown in FIG. 3, the top panels of each section (A), (B) and (C) show the peripheral waveforms recorded from LabChart in the time domain, and the bottom panels of each section show the Fourier transformation. Specifically, in the Fourier transformation charts, there are distinct peaks at frequencies $F_0$ and $F_1$, where $F_0$ represents the porcine's respiratory rate and its contribution to the signal, and $F_1$ represents the porcine's heart rate. The amplitude of the signal was correlated directly with the volume status.

Hemorrhage and Autologous Blood Transfusion

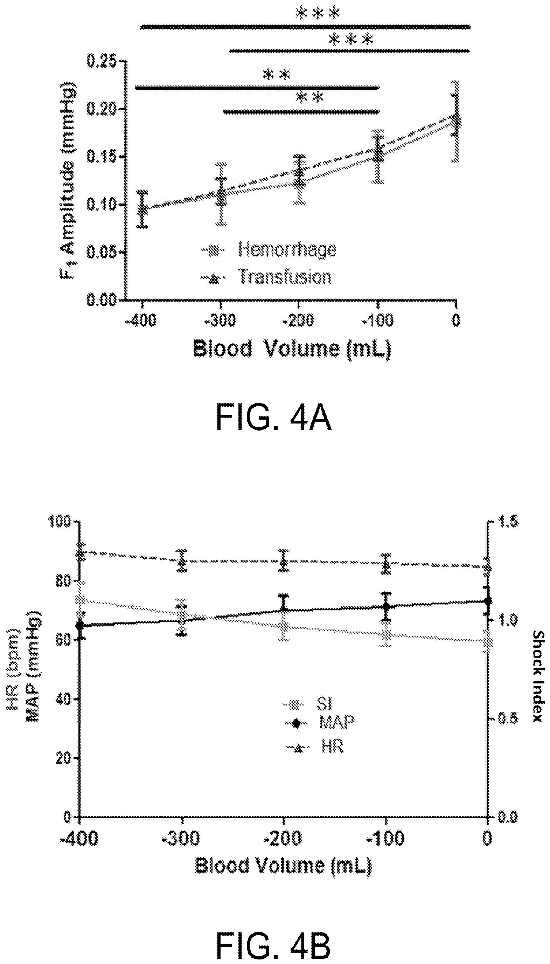

FIG. 4A shows a chart of the $F_1$ amplitude of the hemorrhage and transfusion of blood in the porcine model for volume status according to certain embodiments of the present invention. As shown in FIG. 4A, blood loss of of 200, 300, and 400 mL of crystalloid created significant differences in the $F_1$ amplitude (P<0.05). When measuring the $F_1$ amplitude alone, both the hemorrhage and autologous blood transfusion resulted in significantly non-zero slopes of -0.000223 and -0.000242 and with P values of 0.0049 and 0.0008, respectively. There was a significant change in $F_1$ amplitude following 200 mL (P<0.01), 300 mL (P<0.001), and 400 mL (P<0.001) of blood loss. There was also a significant incremental change in $F_1$ between 100 and 400 mL blood loss.

FIG. 4B shows a chart of mean arterial pressure (MAP), heart rate (HR), and shock index (SI) to the blood volume of the hemorrhage and transfusion of blood in the porcine model for volume status according to certain embodiments of the present invention. Specifically, FIG. 4B shows the arterial pressure, heart rate, and shock index for the pigs over a hemorrhage of 0 to 400 mL. As shown in FIG. 4B, there were no significant differences in the heart rate or the blood pressure after 400 mL blood loss (11.8% estimated blood volume), and the shock index did not significantly change until 300 mL blood loss (8.85% estimated blood volume) (p<0.05).

Taken together, these results show that changes in $F_1$ amplitude is a more sensitive measure for hemorrhage and transfusion of blood than HR, MAP, or SI.

Iatrogenic volume overload

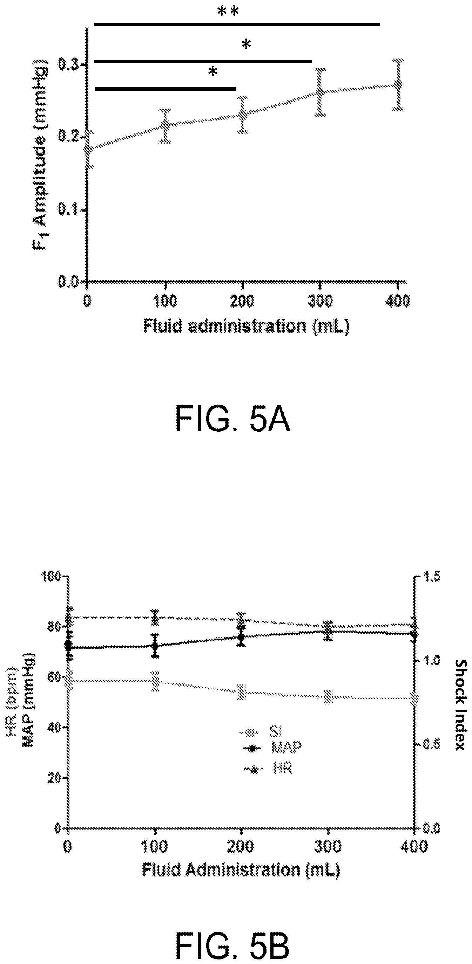

FIG. 5A shows a chart of the $F_1$ amplitude of fluid administration to mild hypervolemia in the porcine model for volume status according to certain embodiments of the present invention. Specifically, FIG. 5A shows the $F_1$ amplitude and how it is affected by additional IV fluid being given. As shown in FIG. 5A, the fluid administration of crystalloid beyond the euvolumic state resulted in significant increase in the $F_1$ amplitude of the signal. Specifically, the $F_1$ amplitude changed with fluid administration resulting in a significantly nonzero slope (p = 0.0017). Additions of 200, 300, and 400 mL of crystalloid created significant differences in the $F_1$ amplitude (P<0.05), between increments of 0 and 200 (P<0.05), 0 and 300 (P<0.05), and 0 and 400 (P<0.01) mL of crystalloid.

FIG. 5B shows a chart of MAP, HR, and SI to the blood volume of fluid administration to mild hypervolemia in the porcine model for volume status according to certain embodiments of the present invention. Specifically, FIG. 5B shows the HR, MAP and SI for the pigs over volume status ranging from 0 to +400mL. As shown in FIG. 5B, there were no significant changes in the heart rate and MAP with volume overload. Shock index changed significantly between 0 and 300 mL (P<0.05) of fluid administration and 0 and 400 mL of fluid administration (P<0.05).

Taken together, these results show that changes in $F_1$ amplitude is a more sensitive measure for hypervolemia than HR, MAP, or SI.

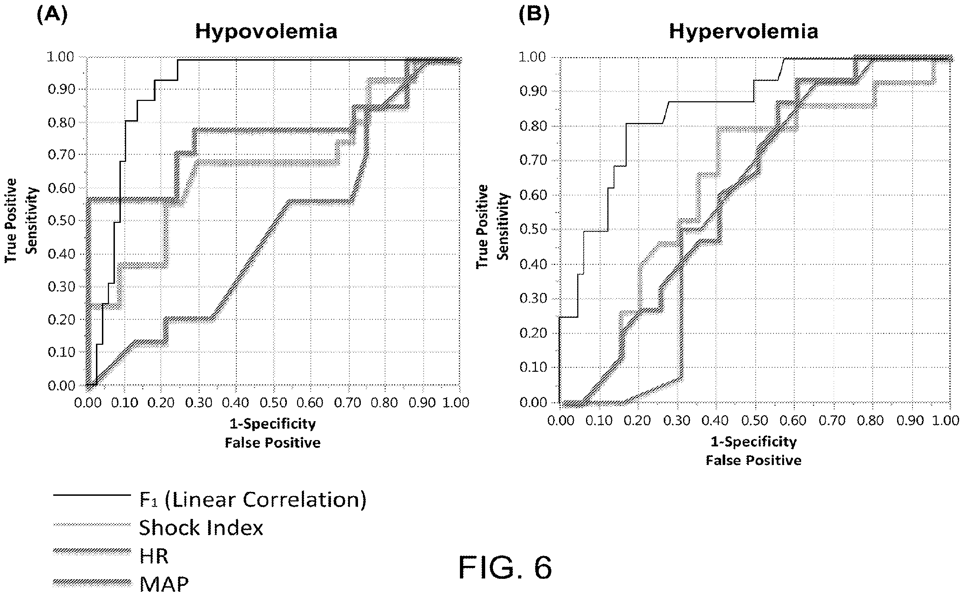

Further, HR, MAP, SI and $F_1$ amplitude were measured during hemorrhage and fluid administration to the porcine volume status model. FIG. 6 shows the receiver operator curves (ROC) for detection of (A) hypovolemia (>200mL hemorrhage, 5.9%) and (B) hypervolemia (>200mL fluid administration, 5.9%) for the peripheral venous signal, HR, MAP, and SI according to certain embodiments of the present invention. As shown in FIG. 6, measurement of $F_1$ amplitude showed improved sensitivity and specificity for detection of hypovolemia and hypervolemia in the porcine model. For the detection of hypovolemia as shown in FIG. 6(A), the $F_1$ amplitude generated an ROC curve with an area under the curve (AUC) of 0.93, HR generated an ROC curve with an AUC of 0.61, MAP generated an ROC curve with an AUC of 0.48, and SI generated an ROC curve with an AUC of 0.72. For the detection of hypervolemia as shown in FIG. 6(B), the $F_1$ amplitude generated an ROC curve with an area under the curve (AUC) of 0.85, HR generated an ROC curve with an AUC of 0.62, MAP generated an ROC curve with an AUC of 0.63, and SI generated an ROC curve with an AUC of 0.65. In sum, MAP demonstrated the weakest ROC curves for the hypovolemia, and HR was the least sensitive at detecting hypervolemia in the porcine model. In all situations the $F_1$ amplitude obtained by PIVA had the greatest sensitivity and specificity at detecting volume status.

The test results are in agreement with findings of Sileshi, et al. which demonstrated hemorrhage detection after approximately -6% estimated blood loss in a cardiac surgery population [31]. The ROCs illustrate that PIVA had a significantly greater sensitivity and specificity for detecting hemorrhage compared to HR, MAP, and SI, as shown in FIG. 2B. Further, PIVA is measured independently of intrathoracic pressure changes produced by mechanical ventilation and may be able to fill a void in dynamic monitoring. [16, 17]

Further, the significant changes in the $F_1$ amplitude has been demonstrated not only during hemorrhage, but also a return of the $F_1$ amplitude to baseline with transfusion of a matched quantity of autologous blood during resuscitation, as shown in FIGS. 4A and 4B. Detecting a return to euvolemia has great potential to enhance accurate goal directed volume resuscitation to prevent fluid overload in the hemorrhaging patient.

In addition, the test simulated iatroagenic volume overload with balanced crystalloid, an inadvertent, yet common event during acute resuscitation. PIVA detected iatrogenic volume overload following only 200mL (5.9% estimated total blood volume) of crystalloid administration beyond the euvolemic state, as shown in FIGS. 5A and 5B. ROC showed PIVA was significantly more sensitive and specific in detecting hypervolemia as compared to SI, HR, and MAP, as shown in FIG. 6. With fluid administration beyond the euvolemic state, the $F_1$ amplitude significantly increases, which provides a novel method for detecting and quantifying fluid overload. This is likely because the additional fluid administration increased wall tension in the vein, thereby increasing the fundamental frequency amplitude.

## EXAMPLE 2

**TEST IN CONTROLLED HUMAN HEMORRHAGIC MODEL**

In this example, a test has been performed using PIVA in a controlled human hemorrhagic model to analyze dynamic changes in the peripheral venous waveforms to assess volume status. The test was approved by the Vanderbilt University Institutional Review Board (IRB), and informed written consent was obtained preoperatively in select patients scheduled for elective cardiac surgery. Any patient undergoing elective cardiac surgery met the inclusion criteria. Patients with a history of moderate or severe right ventricular dysfunction, severe anemia (hemoglobin <8 g/dl) or patients who presented with arrhythmias or hemodynamic instability were excluded. A total of 12 patients were studied.

Anesthesia and Mechanical Ventilation

All patients were induced with an opiate and propofol and received non-depolarizing neuromuscular blockade so that there was no evidence of spontaneous respirations. All patients were intubated with an endotracheal tube and received mechanical ventilation with tidal volumes of 7 to 9 mL/kg, positive end-expiratory pressure of 4 mmHg, and a fraction of inspired oxygen of 1.0. Patients were maintained with 0.8 to 1 MAC of isoflurane. In five patients, mechanical ventilation was halted for brief intervals (<10 seconds) for reasons unrelated to this protocol, such as surgeon's request. This opportunity was used to evaluate the effects of mechanical ventilation on the peripheral venous signal. Comparisons have been conducted between the peripheral venous waveform tracings during breath holding and immediately after mechanical ventilation resumed. The ventilator circuit remained connected to the endotracheal tube throughout this period.

Hemodynamics

All patients were monitored with standard non-invasive monitors including 5-lead electrocardiogram, non-invasive blood pressure cuff, and pulse oximetry. Prior to induction of anesthesia, a 20g arterial catheter (Arrow International, Reading, PA) was inserted into the radial artery. Following induction of anesthesia, all patients received a right 9Fr central catheter (Cook Critical Care, Bloomington, IN), and a pulmonary artery catheter (Edwards Lifesciences, Irvine, CA). To minimize complications, the pulmonary artery catheter was not placed in the wedge position. The pulmonary artery diastolic pressure was used as a surrogate for pulmonary artery occlusion pressure. All pressure transducers were placed at the level of the right atrium and zeroed to atmospheric pressure.

Peripheral Venous Waveform Acquisition:

After induction of anesthesia, an 18 or 16 gauge peripheral intravenous catheter (IV) was inserted into an upper extremity vein and directly connected to a pressure transducer (ADInstruments, Colorado Springs, CO) via standard high- pressure tubing. The IV catheter was dedicated to obtaining venous waveforms and was not used for infusion of fluids or drug delivery during the study. Peripheral venous waveform tracings were recorded continuously throughout the procedure and analyzed on LabChart (ADInstruments, Colorado Springs, CO). Peripheral intravenous waveform data was captured at 1kHz for adequate sampling to perform spectral analysis on the data.

Hemorrhage Protocol

Following induction of anesthesia and central line placement, the hemorrhage protocol was initiated. Standard IV tubing was attached proximally to the central venous catheter and distally to a citrate bag, producing a closed system. Venous blood was removed from the central venous catheter via gravity over a 10-minute period. Up to 500ml or 10% of blood volume was removed from each patient. All were successfully completed within 10 minutes without protocol interruption. Vital signs including heart rate, arterial blood pressure, central venous pressure, pulmonary artery pressure, and PVA were continuously recorded throughout the procedure.

Spectral Analysis

The spectral FFT analysis of peripheral venous pressure was performed using an 8K sampling window with no window overlap. Since data was recorded at a sampling rate of 1 kHz, 8 seconds of continuous time-domain signal was required to perform the 8K-FFT spectral analysis. Once the data is transformed into the frequency domain the amplitude of each frequency is calculated in LabChart. The lowest frequency peak is associated with the patient's respiratory rate ($F_0$) followed by the patient's heart rate ($F_1$). Data was captured in triplicate for each point used in analysis. Peripheral waveform is sensitive to patient movement and electrocautery signal. To minimize signal noise, data was captured during periods of minimal patient movement and no electrocautery use.

Statistical analysis:

Data for physiologic measurements and their associated blood loss was analyzed using a one-way ANOVA analysis with a post-test of Tukey's multiple comparison with paired analysis. Data was also entered as baseline measurements and blood loss of 500 mL in patients into MedCalc to determine a pairwise comparison of ROC curves. Area under the curve, standard error, and a 95% confidence interval for each data set was acquired. ROC curves were plotted on the same graph to easily identify differences between the sensitivity and specificity of physiologic parameters. To determine the effect of positive pressure ventilation, $F_0$ and $F_1$ values were compared using a paired student's t-test with values of $P<0.05$ being considered statistically significant. Statistical analysis was performed using GraphPad Prism and MedCalc.

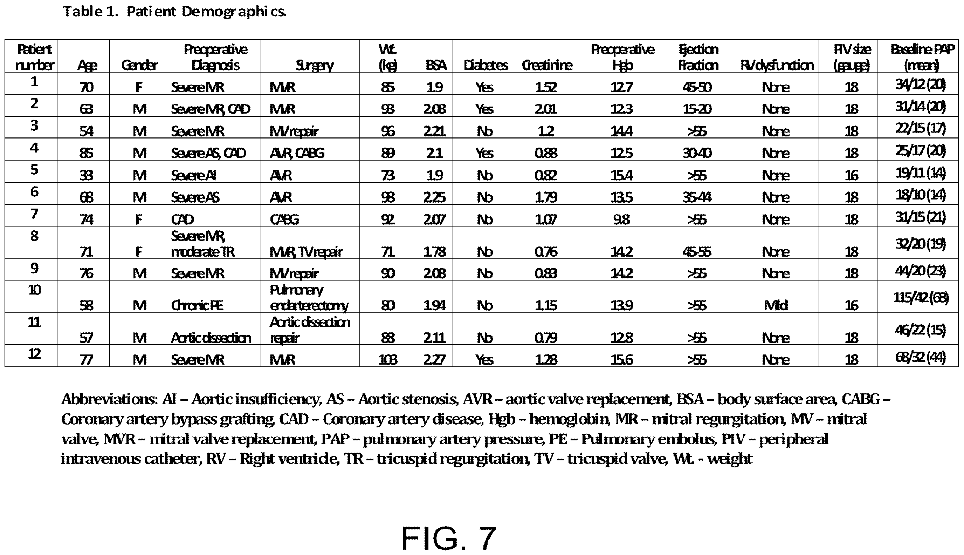

FIG. 7 shows a table of patient demographics in a controlled human hemorrhagic model according to certain embodiments of the present invention. As shown in FIG. 7, twelve patients who underwent elective cardiac surgery were enrolled in this test. Mean age of the patients was 65.5 years, with 9 (75%) being male. The patients underwent variety of cardiac surgical procedures with 6 of 12 (50%) of patients presenting for mitral valve repair/replacement for mitral regurgitation. All patients with the exception of one had normal right ventricular function. Seven patients (58%) presented with a normal left ventricular ejection fraction (LVEF); two patients (16%) had a mild LVEF depression; two patients (16%) had a moderate LVEF depression; and one (8%) patient had a severely depressed LVEF of 15-20%. One patient had severe pulmonary hypertension from chronic pulmonary emboli. In 10 patients, autologous blood donation was completed prior to sternal incision. In four patients, autologous blood was removed following a right thoracotomy for minimally invasive mitral surgery. These patients remained on two-lung ventilation throughout the protocol.

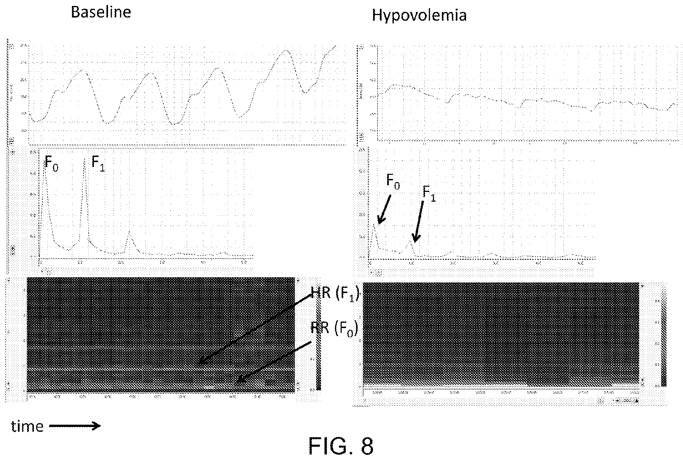

FIG. 8 shows charts of the peripheral venous waveform and the Fourier transformation of the signals at baseline and after autologous blood removal in the controlled human hemorrhagic model according to certain embodiments of the present invention. As shown in FIG. 8, the top panels show the peripheral waveforms, and the bottom panels show the corresponding Fourier transformation of data at baseline and after blood removal. There was a notable amplitude decrease in $F_0$, $F_1$, and higher frequencies following hemorrhage.

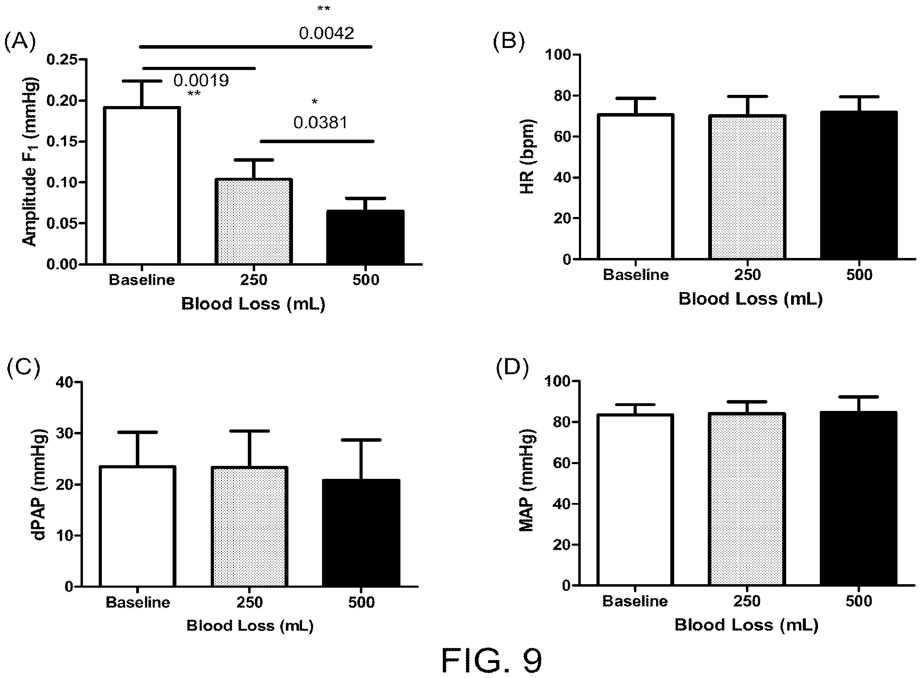

FIG. 9 shows charts of hemodynamic measurements for (A) the $F_1$ amplitude, (B) HR, (C) diastolic pulmonary artery pressure (dPAP) and (D) MAP in the controlled human hemorrhagic model at baseline and following hemorrhage at 250 mL and 500mL according to certain embodiments of the present invention. Specifically, FIG. 9 is provided for a comparison of different indices of volume status during blood loss. As shown in FIG. 9, there were statistically significant changes in the $F_1$ amplitude with blood removal between baseline (0mL) and 250mL (p=0.0019), baseline and 500mL (p=0.0042), and 250mL to 500mL (p=0.0382). MAP, HR, and dPAP did not significantly change between baseline and 250mL or 500mL blood volume removal.

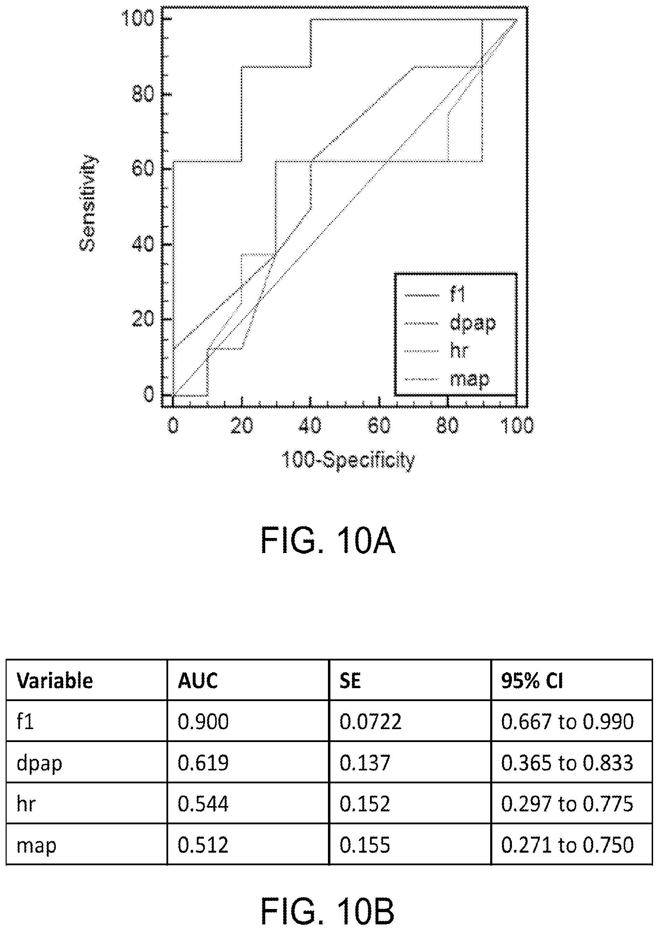

FIG. 10A shows the ROC curves for detection of the $F_1$ amplitude, dPAP, HR and SI according to certain embodiments of the present invention, and FIG. 10B shows a table of the area under the curve (AUC), standard error (SE) and 95% confidence interval for the data as shown in FIG. 10A according to certain embodiments of the present invention. As shown in FIG. 8A, at 6% estimated blood loss, $F_1$ had the greatest area under the ROC curve (AUC = 0.90, 95%CI [0.67, 0.99]), compared to dPAP (AUC = 0.62), heart rate (AUC = 0.54) and MAP (AUC = 0.512).

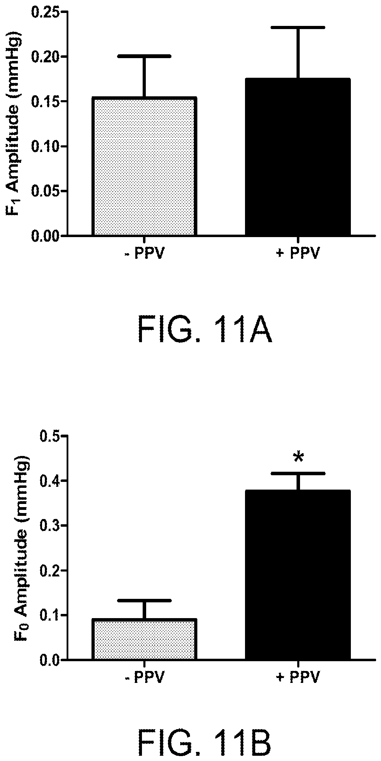

In order to demonstrate independence of $F_1$ signal from effects of positive pressure ventilation, the $F_1$ and $F_0$ amplitudes are recorded with and without mechanical ventilation. FIG. 11A shows the $F_1$ amplitudes with (+PPV) and without (-PPV) positive pressure ventilation according to certain embodiments of the present invention, and FIG. 11B shows the $F_0$ amplitudes with (+PPV) and without (-PPV) positive pressure ventilation according to certain embodiments of the present invention. As shown in FIG. 11A, there was no significant change in the $F_1$ amplitude with and without mechanical ventilation (n=5, P=0.21). In comparison, the $F_0$ amplitude, which represents the respiratory frequency as reflected by changes in the peripheral venous waveform, was significantly greater in ventilated patients compared to non-ventilated patients (n=5, P=0.0059), as shown in FIG. 11B.

Based on the test results, PIVA is able to detect as little as 6% blood volume loss, and the signal was independent of mechanical ventilation. This amount of blood loss is well within the definition of Stage I or subclinical hemorrhage. In contrast, heart rate, MAP, CVP, and dPAP were poor predictors of early stage hemorrhage, a finding that is consistent with previously published studies [39] [6] [40].

## EXAMPLE 3

## ADDITIONAL TESTS

As shown in the previous examples, the inventors have found that venous waveform analysis overcomes many critical barriers associated with arterial-based monitoring. The inventors discovered and confirmed with tests that peripheral intravenous waveform analysis (PIVA) obtained via a pressure transducer in a standard intravenous catheter detects hemorrhage in humans and porcine models.

The inventors have conducted additional tests on PIVA detections of blood loss in pigs: In the test, the PIVA device is applied to intubated and sedated pigs (n=4). All pigs were monitored in real time with intra-arterial blood pressure, heart rate, pulse oximeter, and a 5-lead electrocardiogram. The PIVA device was interfaced with LabChart (ADInstruments, Colorado Springs, CO, USA) software for continuous, real-time data collection. Up to 15% of blood volume was incrementally removed during a 20-minute period.

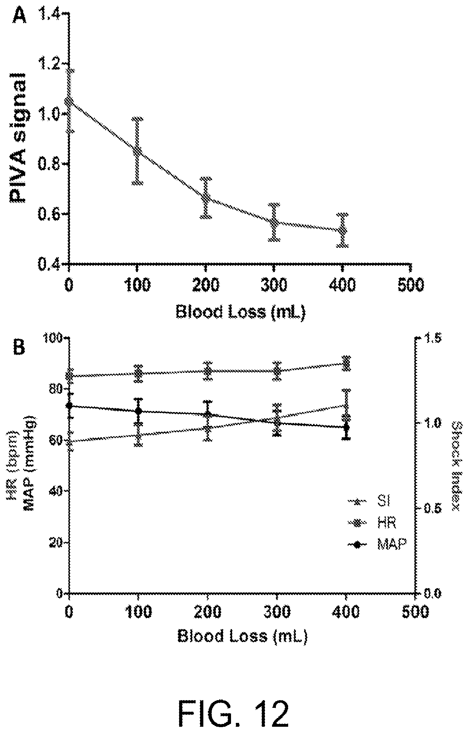

FIG. 12 shows (A) PIVA signal and (B) shock index for detecting hemorrhage in a porcine animal model (n=8), according to certain embodiments of the present invention. As shown in FIG. 12, PIVA waveform data analyzed with Fourier transformation techniques was found to be more sensitive than invasive arterial blood pressure, heart rate, and even shock index for detecting hemorrhage and determining fluid status.

Fluid overload is responsible for increased mortality in injured patients. However, standard vital sign monitoring fails to detect thresholds for euvolemia and hypervolemia, contributing to unnecessary and potentially harmful over-resuscitation. We studied a porcine animal resuscitation model to determine intravascular volume status during fluid administration. Animals were considered euvolemic upon presentation based on weight and hemodynamic stability. After completion of the hemorrhagic stage, pigs (n=8) underwent fluid resuscitation. Autologous blood was re-administered followed by an additional 10 mL/kg of balanced crystalloid solution over 20 minutes.

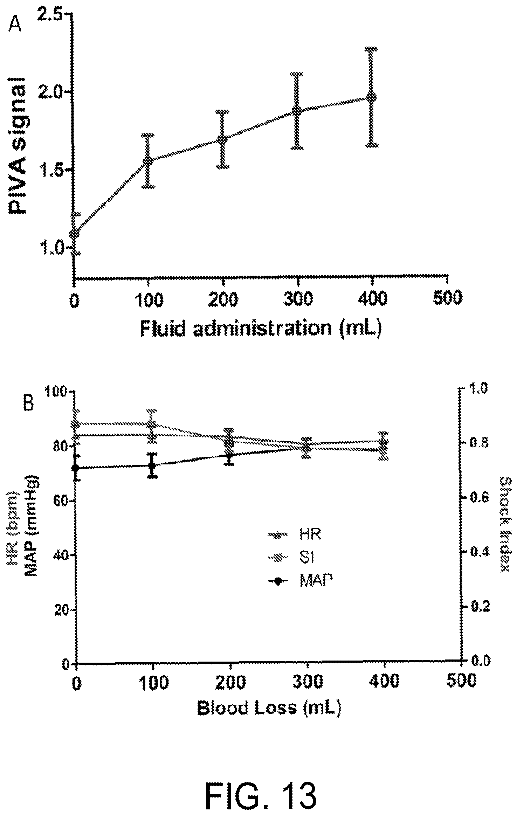

FIG. 13 shows (A) PIVA signal and (B) shock index for detecting hemorrhage in a porcine animal model (n=8), according to certain embodiments of the present invention. As shown in FIG. 13, PIVA continues to detect intravascular changes beyond the euvolemic state during fluid administration in a porcine model n=8 pigs. Notably, heart rate and blood pressure remained unchanged even after 10 mL/kg of crystalloid administration beyond the euvolemic state (data not shown). It is shown from these data that hypervolemia detection with PIVA is superior to standard and invasive monitoring for guiding resuscitation. Development of an algorithm for detecting fluid overload is an extension of the method. In addition to resuscitation, these methods may be useful for managing patients with congestive heart failure.

In summary, the ability of PIVA to detect subclinical hemorrhage and goal-directed fluid therapy offers significant advantages over standard and possibly dynamic monitoring modalities. Further, PIVA is minimally invasive, requires minimal training, and it averts infectious and major vascular complications associated with central venous and pulmonary artery catheters. The PIVA signal is independent of effects of mechanical ventilation and may be useful in in patients receiving lung protective ventilator strategies and possibly in spontaneously breathing individuals. PIVA has the potential to provide a low-cost, minimally invasive method for detecting and quantitating subclinical hemorrhage. As shown in the examples, PIVA is more sensitive than standard and invasive monitoring for detecting subclinical hemorrhage, and may provide a powerful alternative to central venous catheterization. Further, the method is independent of effects of mechanical ventilation, a potential advancement for monitoring spontaneously breathing patients.

The foregoing description of the exemplary embodiments of the invention has been presented only for the purposes of illustration and description and is not intended to be exhaustive or to limit the invention to the precise forms disclosed. Many modifications and variations are possible in light of the above teaching.

The embodiments were chosen and described in order to explain the principles of the invention and their practical application so as to enable others skilled in the art to utilize the invention and various embodiments and with various modifications as are suited to the particular use contemplated. Alternative embodiments will become apparent to those skilled in the art to which the present invention pertains without departing from its scope. Accordingly, the scope of the present invention is defined by the appended claims rather than the foregoing description and the exemplary embodiments described therein.

## LISTING OF REFERENCES

[1]. Wilson, M., D.P. Davis, and R. Coimbra, Diagnosis and monitoring of hemorrhagic shock during the initial resuscitation of multiple trauma patients: a review. The Journal of Emergency Medicine, 2003. 24(4): p. 413-422.
[2]. Payen, D., et al., A positive fluid balance is associated with a worse outcome in patients with acute renal failure.

Crit Care, 2008. 12(3): p. R74.

[3]. Wiedemann, H.P., et al., Comparison of two fluid-management strategies in acute lung injury. N Engl J Med, 2006. 354(24): p. 2564-75.

[4]. Bouchard, J., et al., Fluid accumulation, survival and recovery of kidney function in critically illpatients with acute kidney injury. Kidney Int, 2009. 76(4): p. 422-7.

[5]. Edelman, D.A., et al., Post-traumatic hypotension: should systolic blood pressure of 90-109 mmHg be included? Shock, 2007. 27(2): p. 134-8.

[6]. Kumar, A., et al., Pulmonary artery occlusion pressure and central venous pressure fail to predict ventricular filling volume, cardiac performance, or the response to volume infusion in normal subjects. Crit Care Med, 2004. 32(3): p. 691-9.

[7]. Mermel, L., Infections related to central venous catheters in US intensive-care units. Lancet, 2003. 361(9368): p. 1562.

[8]. Garland, A. and A.F. Connors, Indwelling arterial catheters in the intensive care unit: necessary and beneficial, or a harmful crutch? Am J Respir Crit Care Med, 2010. 182(2): p. 133-4.

[9]. Domino, K.B., et al., Injuries and liability related to central vascular catheters: a closed claims analysis. Anesthesiology, 2004. 100(6): p. 1411-1418.

[10]. Bendjelid, K. and J. Romand, Fluid responsiveness in mechanically ventilated patients: a review of indices used in intensive care. Intensive Care Medicine, 2003. 29(3): p. 352-360.

[11]. Eyre, L. and A. Breen, Optimal volaemic status and predicting fluid responsiveness. Continuing Education in Anaesthesia, Critical Care & Pain, 2010. 10(2): p. 59-62.

[12]. Marik, P., Hemodynamic Parameters to Guide Fluid Therapy. Transfusion Alternatives in Transfusion Medicine, 2010. 11(3): p. 102-112.

[13]. Charalambous, C., et al., Comparison of peripheral and central venous pressures in critically Illpatients. Anaesth Intensive Care, 2003. 31(1): p. 34-9.

[14]. Charalambous, C., et al., Comparison of peripheral and central venous pressures in critically illpatients. Anaesthesia and Intensive Care, 2003. 31(1): p. 34-39.

[15]. Pikwer, A., et al., Fluid balance monitoring by cuff-occluded rate of rise of peripheral venous pressure in haemodialysis patients. Anaesthesia, 2012. 67(8): p. 894-8.

[16]. Alian, A.A., et al., Impact of lower body negative pressure induced hypovolemia on peripheral venous pressure waveform parameters in healthy volunteers. Physiol Meas, 2014. 35(7): p. 1509-20.

[17]. Sileshi, B., et al., Peripheral venous waveform analysis for detecting early hemorrhage: a pilot study. Intensive Care Med, 2015. 41(6): p. 1147-8.

[18]. Kutcher, M.E., et al., A paradigm shift in trauma resuscitation: evaluation of evolving massive transfusion practices. JAMA Surg, 2013. 148(9): p. 834-40.

[19]. Huang, Q., et al., Fluid volume overload negatively influences delayed primary facial closure in open abdomen management. J Surg Res, 2014. 187(1): p. 122-7.

[20]. Group, T.A.I.a.t.A.C.T., Goal-Directed Resuscitation for Patients with Early Septic Shock. N Engl J Med, 2014.

[21]. Blow, O., et al., The golden hour and the silver day: detection and correction of occult hypoperfusion within 24 hours improves outcome from major trauma. J Trauma, 1999. 47(5): p. 964-9.

[22]. Desebbe, O. and M. Cannesson, Using ventilation-induced plethysmographic variations to optimize patient fluid status. Curr Opin Anaesthesiol, 2008. 21(6): p. 772-8.

[23]. De Backer, D., et al., Pulse pressure variations to predict fluid responsiveness: influence of tidal volume. Intensive Care Med, 2005. 31(4): p. 517-23.

[24]. Auler, J.O., Jr., et al., Online monitoring of pulse pressure variation to guide fluid therapy after cardiac surgery. Anesth Analg, 2008. 106(4): p. 1201-6, table of contents.

[25]. Berkenstadt, H., et al., Pulse pressure and stroke volume variations during severe haemorrhage in ventilated dogs. Br J Anaesth, 2005. 94(6): p. 721-6.

[26]. Graham, M.R., K. McCrea, and L.G. Girling, Pulse pressure variability during hemorrhage and reinfusion in piglets: effects of age and tidal volume. Can J Anaesth, 2014. 61(6): p. 533-42.

[27]. Yang, X. and B. Du, Does pulse pressure variation predict fluid responsiveness in critically illpatients? A systematic review and meta-analysis. Crit Care, 2014. 18(6): p. 650.

[28]. Oliveira-Costa, C.D., et al., Pulse pressure variation and prediction of fluid responsiveness in patients ventilated with low tidal volumes. Clinics (Sao Paulo), 2012. 67(7): p. 773-8.

[29]. Marik, P.E., X. Monnet, and J.L. Teboul, Hemodynamic parameters to guide fluid therapy. Ann Intensive Care, 2011. 1(1):p. 1.

[30]. Heenen, S., D. De Backer, and J.L. Vincent, How can the response to volume expansion in patients with spontaneous respiratory movements be predicted? Crit Care, 2006. 10(4): p. R102.

[31]. Sileshi, B., et al., Peripheral venous waveform analysis for detecting early hemorrhage: a pilot study. Intensive Care Med, 2015.

[32]. Cannon, C.M., et al., Utility of the shock index in predicting mortality in traumatically injured patients. J Trauma, 2009. 67(6): p. 1426-30.

[33]. Holte, K., N. Sharrock, and H. Kehlet, Pathophysiology and clinical implications of perioperative fluid excess. British Journal of Anaesthesia, 2002. 89(4): p. 622-632

[34]. Mabry, R.L., et al., Impact of critical care-trained flight paramedics on casualty survival during helicopter evacuation in the current war in Afghanistan. J Trauma Acute Care Surg, 2012. 73(2 Suppl 1): p. S32-7.

[35]. Parks, J.K., et al., Systemic hypotension is a late marker of shock after trauma: a validation study of Advanced Trauma Life Support principles in a large national sample. The American Journal of Surgery, 2006. 192(6): p. 727-731.

[36]. Vincent, J.L., et al., Clinical review: Update on hemodynamic monitoring--a consensus of 16. Crit Care, 2011. 15(4): p. 229.

[37]. Marik, P.E., X. Monnet, and J.L. Teboul, Hemodynamic parameters to guide fluid therapy. Ann Intensive Care, 2011. 1(1): p. 1.

[38]. Desebbe, O. and M. Cannesson, Using ventilation-induced plethysmographic variations to optimize patient fluid status. Curr Opin Anaesthesiol, 2008. 21(6): p. 772-8.

[39]. Wheeler, A.P., et al., Pulmonary-artery versus central venous catheter to guide treatment of acute lung injury. N Engl J Med, 2006. 354(21): p. 2213-24.

[40]. Buhre, W., et al., Changes in central venous pressure and pulmonary capillary wedge pressure do not indicate changes in right and left heart volume in patients undergoing coronary artery bypass surgery. Eur J Anaesthesiol, 1999. 16(1): p. 11-7.

[41]. Holcomb JB, McMullin NR, Pearse L, et al. Causes of death in U.S. Special Operations Forces in the global war on terrorism: 2001-2004. Annals of surgery 2007;245:986-91.

[42]. Eastridge BJ, Hardin M, Cantrell J, et al. Died of wounds on the battlefield: causation and implications for improving combat casualty care. The Journal of trauma 2011;71:S4-8.

[43]. Swaroop M, Straus DC, Agubuzu O, Esposito TJ, Schermer CR, Crandall ML. Pre-hospital transport times and survival for Hypotensive patients with penetrating thoracic trauma. Journal of emergencies, trauma, and shock 2013;6:16-20.

[44]. Riskin DJ, Tsai TC, Riskin L, et al. Massive transfusion protocols: the role of aggressive resuscitation versus product ratio in mortality reduction. Journal of the American College of Surgeons 2009;209:198-205.

[45]. Duke MD, Guidry C, Guice J, et al. Restrictive fluid resuscitation in combination with damage control resuscitation: time for adaptation. The journal of trauma and acute care surgery 2012;73:674-8.

[46]. Paladino L, Sinert R, Wallace D, Anderson T, Yadav K, Zehtabchi S. The utility of base deficit and arterial lactate in differentiating major from minor injury in trauma patients with normal vital signs. Resuscitation 2008;77:363-8.

[47]. Convertino VA. Blood pressure measurement for accurate assessment of patient status in emergency medical settings. Aviation, space, and environmental medicine 2012;83:614-9.

[48]. Convertino VA, Ryan KL, Rickards CA, et al. Physiological and medical monitoring for en route care of combat casualties. The Journal of trauma 2008;64:S342-53.

[49]. Gutierrez G, Reines HD, Wulf-Gutierrez ME. Clinical review: hemorrhagic shock. Critical care 2004;8:373-81.

[50]. Cocchi MN, Kimlin E, Walsh M, Donnino MW. Identification and resuscitation of the trauma patient in shock. Emergency medicine clinics of North America 2007;25:623-42, vii.

[51]. Marik PE, Cavallazzi R, Vasu T, Hirani A. Dynamic changes in arterial waveform derived variables and fluid responsiveness in mechanically ventilated patients: a systematic review of the literature. Critical care medicine 2009;37:2642-7.

[52]. Teboul JL, Monnet X. Prediction of volume responsiveness in critically ill patients with spontaneous breathing activity. Current opinion in critical care 2008;14:334-9.

## Claims

1. A peripheral intravenous (IV) waveform analysis (PIVA) system, comprising:

   a peripheral IV device (110) configured to acquire, from a vein of a living subject, peripheral venous signals; and
   a processing device (120) communicatively connected to the peripheral IV device, configured to:

   receive (S210) the peripheral venous signals from the peripheral IV device; and
   perform (S220) a spectral analysis on the peripheral venous signal to obtain a peripheral venous pressure frequency spectrum;

**characterized in that** the processing device is further configured to:
perform (S230) a statistical analysis on amplitudes of peaks of the peripheral venous pressure frequency spectrum to determine an intravascular volume status of the living subject in real time.

2. The PIVA system of claim 1, wherein:

   the intravascular volume status of the living subject indicates hypovolemia when amplitude decreases greater than a first threshold are detected from the peaks of the peripheral venous pressure frequency spectrum; and
   the intravascular volume status of the living subject indicates hypervolemia when amplitude increases greater than a second threshold are detected from the peaks of the peripheral venous pressure frequency spectrum.

3. The PIVA system of claim 1, being applied to the living subject during resuscitation of the living subject, wherein:

   the intravascular volume status of the living subject indicates a return of euvolemia from a hypovolemic state when the living subject is determined to be in the hypovolemic state at an earlier time period, and amplitude increases greater than a third threshold are detected from the peaks of the peripheral venous pressure frequency spectrum; and
   the intravascular volume status of the living subject indicates over-resuscitation when the living subject is determined to be in an euvolemic state at the earlier time period, and amplitude increases greater than a fourth threshold are detected from the peaks of the peripheral venous pressure frequency spectrum;
   wherein the processing device is further configured to detect efficacy of treatment and the return to euvolemia in the living subject based on the intravascular volume status of the living subject.

4. The PIVA system of claim 1, wherein the processing device is communicatively connected to the peripheral IV device through a wireless connection, and the peripheral IV device comprises:

   a peripheral IV catheter being inserted into the vein of the living subject; and
   a monitoring device connected to the peripheral IV catheter, configured to capture and record the peripheral venous signals from the peripheral IV catheter at a sampling rate, wherein the monitoring device comprises a pressure transducer directly connected to the peripheral IV catheter, wherein the peripheral venous signals are captured and recorded by the pressure transducer.

5. The PIVA system of claim 1, further comprises:

   a pump connected to the living subject to perform liquid exchange to the living subject, wherein the pump is a dialysis pump, a cardiopulmonary bypass pump, an extracorporeal membrane oxygenation (ECMO), or an infusion pump; and
   a pump controlling mechanism communicatively connected to the processing device, configured to control the pump by intermittently pausing the pump or subtract the pump signal when the peripheral IV device acquires the peripheral venous signals, and restarting the pump when the peripheral IV device does not acquire the peripheral venous signals;
   wherein the processing device is further configured to send a signal to the pump controlling mechanism to notify the pump controlling mechanism to control the pump.

6. The PIVA system of claim 2, configured to be used on the living subject during ultrafiltration/dialysis or diuresis of the living subject, wherein the processing device (120) is further configured to:
   generate an alert message when the intravascular volume status of the living subject indicates hypovolemia.

7. The PIVA system of claim 1, wherein the spectral analysis is a spectral fast Fourier transform (FFT) analysis, wherein the statistical analysis comprises:

   obtaining a plurality of baseline peaks $\{B_{N-1}\}$ on a baseline peripheral venous pressure frequency spectrum, wherein N is a positive integer, and the plurality of baseline peaks $\{B_{N-1}\}$ respectively corresponds to a plurality of frequencies $\{F_0, F_1, ..., F_N\}$, such that $B_{N-1}$ is a function of $F_{N-1}$ satisfying $B_{N-1} = B_{N-1} (F_{N-1})$, wherein $F_N$ is greater than $F_{N-1}$;
   obtaining a plurality of peaks $\{P_{N-1}\}$ on the peripheral venous pressure frequency spectrum, wherein the plurality of peaks $\{P_{N-1}\}$ correspond to the plurality of frequencies $\{F_0, F_1, ..., F_N\}$, such that $P_{N-1}$ is a function of $F_{N-1}$ satisfying $P_{N-1} = P_{N-1} (F_{N-1})$; and

determining the intravascular volume status of the living subject in real time by comparing the amplitudes of the peaks $\{P_{N-1}\}$ to that of the baseline peaks $\{B_{N-1}\}$ respectively, wherein

the plurality of peaks $\{P_{N-1}\}$ comprises a first peak $P_0$ corresponding to a first frequency $F_0$ and a second peak $P_1$ corresponding to a second frequency $F_1$;
the first peak $P_0$ corresponding to the first frequency $F_0$ is associated with a respiratory rate of the living subject; and
the second peak $P_1$ corresponding to the second frequency $F_1$ is associated with a heart rate of the living subject;

wherein the baseline peripheral venous pressure frequency spectrum is obtained by:

acquiring the peripheral venous signals from the vein of the living subject at an earlier time period; and
processing the peripheral venous signals acquired at the earlier time period by the spectral FFT analysis to obtain the baseline peripheral venous pressure frequency spectrum.

8. The PIVA system of claim 1, for determining hypovolemia, hypervolemia and vascular tone of the living subject based on the intravascular volume status of the living subject,wherein

the peripheral IV device (110) is further configured to acquire (S210), continuously for a time period from $T_0$ to $T_2$, the peripheral venous signals from the vein of the living subject, wherein the time period is divided into a first time period from $T_0$ to $T_1$, and a second time period from $T_1$ to $T_2$; and
the processing device (120) is further configured to:

process the peripheral venous signals acquired at the first time period to obtain a baseline of the peripheral venous pressure frequency spectrum;
wherein performing the statistical analysis comprises:

obtaining a plurality of baseline peaks $\{B_{N-1}\}$ on the baseline peripheral venous pressure frequency spectrum, wherein N is a positive integer, and the plurality of baseline peaks $\{B_{N-1}\}$ respectively corresponds to a plurality of frequencies $\{F_0, F_1, ..., F_N\}$, such that $B_{N-1}$ is a function of $F_{N-1}$ satisfying $B_{N-1} = B_{N-1}(F_{N-1})$, wherein $F_N$ is greater than $F_{N-1}$;
processing (S220) the peripheral venous signals acquired at the second time period to obtain a peripheral venous pressure frequency spectrum;
obtaining a plurality of peaks $\{P_{N-1}\}$ on the peripheral venous pressure frequency spectrum, wherein the plurality of peaks $\{P_{N-1}\}$ correspond to the plurality of frequencies $\{F_0, F_1, ..., F_N\}$, such that $P_{N-1}$ is a function of $F_{N-1}$ satisfying $P_{N-1} = P_{N-1}(F_{N-1})$; and
determining (S230, S240) the intravascular volume status of the living subject at the second time period by comparing amplitudes of the peaks $\{P_{N-1}\}$ to that of the baseline peaks $\{B_{N-1}\}$ respectively,
wherein the intravascular volume status of the living subject at the second time period indicates hypovolemia or hypervolemia (S250) when amplitude changes greater than a threshold are detected from the baseline peaks $\{B_{N-1}\}$ to the peaks $\{P_{N-1}\}$. $_1\}$.

9. The system of claim 8, wherein:

the intravascular volume status of the living subject at the second time period indicates hypovolemia when amplitude decreases are detected greater than a first threshold from the baseline peaks $\{B_{N-1}\}$ to the peaks $\{P_{N-1}\}$; and
the intravascular volume status of the living subject at the second time period indicates hypervolemia when amplitude increases greater than a second threshold are detected from the baseline peaks $\{B_{N-1}\}$ to the peaks $\{P_{N-1}\}$.

10. The system of claim 8, configured to be used on the living subject during resuscitation of the living subject, wherein:

the intravascular volume status of the living subject at the second time period indicates a return of euvolemia from a hypovolemic state when the living subject is determined to be in the hypovolemic state at the first time period, and amplitude increases greater than a third threshold are detected from the baseline peaks $\{B_{N-1}\}$ to the peaks $\{P_{N-1}\}$; and

the intravascular volume status of the living subject at the second time period indicates over-resuscitation when the living subject is determined to be in an euvolemic state at the first time period, and amplitude increases greater than a fourth threshold are detected from the baseline peaks $\{B_{N-1}\}$ to the peaks $\{P_{N-1}\}$.

11. The system of claim 8, wherein:

the peripheral venous signals are processed by a spectral fast Fourier transform (FFT) analysis to obtain the baseline peripheral venous pressure frequency spectrum and the peripheral venous pressure frequency spectrum, respectively;

the plurality of peaks $\{P_{N-1}\}$ comprises a first peak $P_0$ corresponding to a first frequency $F_0$ and a second peak $P_1$ corresponding to a second frequency $F_1$;

the first peak $P_0$ corresponding to the first frequency $F_0$ is associated with a respiratory rate of the living subject; and

the second peak $P_1$ corresponding to the second frequency $F_1$ is associated with a heart rate of the living subject.

## Patentansprüche

1. Periphere-Intravenöse-(IV)-Wellenform-Analyse-(PIVA)-System, welches aufweist:

eine periphere IV-Vorrichtung (110), die konfiguriert ist, um aus einer Vene eines lebenden Probanden periphere Venensignale zu erfassen; und

eine Prozessvorrichtung (120), die mit der peripheren IV-Vorrichtung (110) kommunikativ verbunden ist, konfiguriert zum

Empfangen (S210) der peripheren Venensignale von der peripheren IV-Vorrichtung; und

Durchführen (S220) einer Spektralanalyse an dem peripheren venösen Signal, um ein Peripherer-Venendruck-Frequenzspektrum zu erhalten;

**dadurch gekennzeichnet, dass** die Prozessvorrichtung ferner konfiguriert ist zum:

Durchführen (S230) einer statistischen Analyse an Amplituden von Peaks des Peripherer-Venendruck-Frequenzspektrums, um einen intravaskulären Volumenstatus des lebenden Probanden in Echtzeit zu bestimmen.

2. Das PIVA-System von Anspruch 1, wobei:

der intravaskuläre Volumenstatus des lebenden Probanden Hypovolämie anzeigt, wenn Amplitudenabnahmen größer als ein erster Schwellenwert aus den Peaks des Peripherer-Venendruck-Frequenzspektrums detektiert werden; und

der intravaskuläre Volumenstatus des lebenden Probanden Hypervolämie anzeigt, wenn Amplitudenanstiege größer als ein zweiter Schwellenwert aus den Peaks des Peripherer-Venendruck-Frequenzspektrums detektiert werden.

3. Das PIVA-System von Anspruch 1, das während Reanimation des lebenden Probanden angewendet wird, wobei:

der intravaskuläre Volumenstatus des lebenden Probanden eine Euvolämie-Rückkehr von einem hypovolämischen Status anzeigt, wenn bestimmt wird, dass der lebende Proband zu einer früheren Zeitspanne im hypovolämischen Status war, und Amplitudenanstiege größer als ein dritter Schwellenwert aus den Peaks des Peripherer-Venendruck-Frequenzspektrums detektiert werden; und

der intravaskuläre Volumenstatus des lebenden Probanden Über-Reanimation anzeigt, wenn bestimmt wird, dass der lebende Proband zu einer früheren Zeitspanne in einem euvolämischen Zustand war, und Amplitudenanstiege größer als ein vierter Schwellenwert aus den Peaks des Peripherer-Venendruck-Frequenzspektrums detektiert werden;

wobei die Prozessvorrichtung ferner konfiguriert ist, um, basierend auf dem intravaskulären Volumenstatus des lebenden Probanden die Effizienz der Behandlung zu detektieren und zur Euvolämie im lebenden Probanden zurückzukehren.

4. Das PIVA-System von Anspruch 1, wobei die Prozessvorrichtung mit der peripheren IV-Vorrichtung durch eine drahtlose Verbindung kommunikativ verbunden ist, und die periphere IV-Vorrichtung aufweist:

einen peripheren IV-Katheter, der in die Vene des lebenden Probanden eingesetzt wird; und

eine Überwachungsvorrichtung, die mit dem peripheren IV-Katheter verbunden ist, konfiguriert, um die peripheren Venensignale von dem peripheren IV-Katheter mit einer Abtastrate aufzunehmen und aufzuzeichnen, wobei die Überwachungsvorrichtung einen Druckwandler aufweist, der direkt mit dem peripheren IV-Katheter verbunden ist, wobei die peripheren Venensignale von dem Druckwandler aufgenommen und aufgezeichnet werden.

5. Das PIVA-System von Anspruch 1, das ferner aufweist:

eine Pumpe, die mit dem lebenden Probanden verbunden ist, um einen Flüssigkeitsaustausch an dem lebenden Probanden durchzuführen, wobei die Pumpe eine Dialysepumpe, eine cardiopulmonäre Bypass-Pumpe, eine Extrakorporale-Membran-Oxygenierungs (ECMO)- oder eine Infusionspumpe ist; und
einen Pumpensteuermechanismus, der mit der Prozessvorrichtung kommunikativ verbunden ist, konfiguriert, um die Pumpe zu steuern durch intermittierendes Pausieren der Pumpe oder Subtrahieren des Pumpensignals, wenn die periphere IV-Vorrichtung die peripheren Venensignale erfasst, und Wiederanlaufen der Pumpe, wenn die periphere IV-Vorrichtung die peripheren Venensignale nicht erfasst;
wobei die Prozessvorrichtung ferner konfiguriert ist, um ein Signal zu dem Pumpensteuermechanismus zu senden, um dem
Pumpensteuermechanismus zu melden, die Pumpe zu steuern.

6. Das PIVA-System von Anspruch 2, das zur Verwendung an dem lebenden Probanden während Ultrafiltration/Dialyse oder Diurese des lebenden Probanden konfiguriert ist, wobei die Prozessvorrichtung (120) ferner konfiguriert ist zum: Erzeugen einer Alarmmeldung, wenn der intravaskuläre Volumenstatus des lebenden Probanden Hypovolämie anzeigt.

7. Das PIVA-System von Anspruch 1, wobei die Spektralanalyse eine schnelle Fourier-Transformations (FFT-)-Analyse ist, wobei die statistische Analyse aufweist:

Erhalten einer Mehrzahl von Grundlinien-Peaks $\{B_{N-1}\}$ auf eines Peripherer-Venendruck-Grundlinien-Frequenzspektrums, wobei N eine positive ganze Zahl ist, und die Mehrzahl von Grundlinien-Peaks $\{B_{N-1}\}$ jeweils einer Mehrzahl von Frequenzen $\{F_0, F_1, ..., F_N\}$ entspricht, so dass $B_{N-1}$ eine Funktion von $F_{N-1}$ ist, die $B_{N-1} = B_{N-1}(F_{N-1})$ erfüllt, wobei FN größer als $F_{N-1}$ ist;
Erhalten einer Mehrzahl von Peaks $\{P_{N-1}\}$ an dem Peripherer-Venendruck-Frequenzspektrum, wobei die Mehrzahl von Peaks $\{P_{N-1}\}$ der Mehrzahl von Frequenzen $\{F_0, F_1, ..., F_N\}$ entspricht, so dass $P_{N-1}$ eine Funktion von $F_{N-1}$ ist, die $P_{N-1} = P_{N-1}(F_{N-1})$ erfüllt; und
Bestimmen des intravaskulären Volumenstatus des lebenden Probanden in Echtzeit durch Vergleichen der jeweiligen Amplituden der Peaks $\{P_{N-1}\}$ mit jenen der Grundlinien-Peaks $\{B_{N-1}\}$, wobei
die Mehrzahl von Peaks $\{P_{N-1}\}$ einen einer ersten Frequenz $F_0$ entsprechenden ersten Peak $P_0$ und einen einer zweiten Frequenz $F_1$ entsprechenden zweiten Peak $P_1$ aufweist;
der der ersten Frequenz $F_0$ entsprechende erste Peak $P_0$ einer Atemfrequenz des lebenden Probanden zugeordnet wird; und
der der zweiten Frequenz $F_1$ entsprechende zweite Peak $P_1$ einem Herzschlag des lebenden Probanden zugeordnet wird;
wobei das Peripherer-Venendruck-Grundlinien-Frequenzspektrum erhalten wird durch:

Erfassen der peripheren Venensignale von der Vene des lebenden Probanden zu einer früheren Zeitspanne; und
Bearbeiten der zu der früheren Zeitspanne erfassten peripheren Venensignale mit der FFT-Spektralanalyse, um das Peripherer-Venendruck-Grundlinien-Frequenzspektrum zu erhalten.

8. Das PIVA-System von Anspruch 1 zum Bestimmen von Hypovolämie, Hypervolämie und Gefäßtonus des lebenden Probanden basierend auf einem intravaskulären Volumenstatus des lebenden Probanden, wobei die periphere IV-Vorrichtung (110) ferner konfiguriert ist, um kontinuierlich für eine Zeitspanne von $T_0$ bis $T_2$ die peripheren Venensignale von der Vene des lebenden Probanden zu erfassen (S210), wobei die Zeitspanne in eine erste Zeitspanne von $T_0$ bis $T_1$ und eine zweite Zeitspanne von $T_1$ bis $T_2$ unterteilt ist; und
die Prozessvorrichtung (120) ferner konfiguriert ist zum:

Bearbeiten der zur ersten Zeitspanne erfassten peripheren Venensignale zum Erhalt einer Grundlinie des Peripherer-Venendruck-Frequenzspektrums;

Wobei das Durchführen der statistischen Analyse aufweist:

Erhalten einer Mehrzahl von Grundlinien-Peaks $\{B_{N-1}\}$ auf dem Peripherer-Venendruck-Grundlinien-Frequenzspektrum, wobei N eine positive ganze Zahl ist, und die Mehrzahl von Grundlinien-Peaks $\{B_{N-1}\}$ jeweils einer Mehrzahl Frequenzen $\{F_0, F_1, ..., F_N\}$ entspricht, so dass $B_{N-1}$ eine Funktion von $F_{N-1}$ ist, die $B_{N-1} = B_{N-1} (F_{N-1})$ erfüllt, wobei FN größer als $F_{N-1}$ ist; Bearbeiten (S220) der zur zweiten Zeitspanne erfassten peripheren Venensignale zum Erhalt eines Peripherer-Venendruck-Frequenzspektrums; Erhalten einer Mehrzahl von Peaks $\{P_{N-1}\}$ an dem Peripherer-Venendruck-Frequenzspektrum, wobei die Mehrzahl von Peaks $\{P_{N-1}\}$ der Mehrzahl von Frequenzen $\{F_0, F_1, ..., F_N\}$ entspricht, so dass $P_{N-1}$ eine Funktion von $F_{N-1}$ ist, die $P_{N-1} = P_{N-1} (F_{N-1})$ erfüllt; und Bestimmen (S230, S240) des intravaskulären Volumentstatus des lebenden Probanden zu der zweiten Zeitspanne durch Vergleichen der jeweiligen Amplituden der Peaks $\{P_{N-1}\}$ mit jenen der Grundlinien-Peaks $\{B_{N-1}\}$, wobei der intravaskuläre Volumenstatus des lebenden Probanden zur zweiten Zeitspanne Hypovolämie oder Hypervolämie (S250) anzeigt, wenn Amplitudenänderungen größer als ein Schwellenwert von den Grundlinien-Peaks $\{B_{N-1}\}$ zu den Peaks $\{P_{N-1}\}$ detektiert werden.

9. Das System von Anspruch 8, wobei:

der intravaskuläre Volumenstatus des lebenden Probanden zu der zweiten Zeitspanne Hypovolämie anzeigt, wenn Amplitudenabnahmen größer als ein erster Schwellenwert von den Grundlinien-Peaks $\{B_{N-1}\}$ zu den Peaks $\{P_{N-1}\}$ detektiert werden; und der intravaskuläre Volumenstatus des lebenden Probanden zu der zweiten Zeitspanne Hypervolämie anzeigt, wenn Amplitudenanstiege größer als ein zweiter Schwellenwert von den Grundlinien-Peaks $\{B_{N-1}\}$ zu den Peaks $\{P_{N-1}\}$ detektiert werden.

10. Das System von Anspruch 8, das zur Verwendung an dem lebenden Probanden während Reanimation des lebenden Probanden konfiguriert ist, wobei:

der intravaskuläre Volumenstatus des lebenden Probanden zur zweiten Zeitspanne eine Rückkehr von einem hypervolämischen Zustand zu Euvolämie anzeigt, wenn bestimmt wird, dass der lebende Proband zu der ersten Zeitspanne in dem hypovolämischen Zustand ist, und Amplitudenanstiege größer als ein dritter Schwellenwert von den Grundlinien-Peaks $\{B_{N-1}\}$ zu den Peaks $\{P_{N-1}\}$ detektiert werden; und der intravaskuläre Volumenstatus des lebenden Probanden zu der zweiten Zeitspanne eine Über-Reanimation anzeigt, wenn bestimmt wird, dass der lebende Proband zu der ersten Zeitspanne in einem euvolämischen Zustand ist, und Amplitudenanstiege größer als ein vierter Schwellenwert von den Grundlinien-Peaks $\{B_{N-1}\}$ zu den Peaks $\{P_{N-1}\}$ detektiert werden.

11. Das System von Anspruch 8, wobei:

die peripheren Venensignale durch eine Schnelle-Fourier-Transformations (FFT-)-Spektralanalyse bearbeitet werden, um jeweils das Peripherer-Venendruck-Grundlinien-Frequenzspektrum und das Peripherer-Venendruck-Frequenzspektrum zu erhalten; die Mehrzahl von Peaks $\{P_{N-1}\}$ einen einer ersten Frequenz $F_0$ entsprechenden ersten Peak $P_0$ und einen einer zweiten Frequenz $F_1$ entsprechenden zweiten Peak $P_1$ aufweist; der der ersten Frequenz $F_0$ entsprechende erste Peak $P_0$ einer Atemfrequenz des lebenden Probanden zugeordnet ist; und der der zweiten Frequenz $F_1$ entsprechende zweite Peak $P_1$ einer Herzfrequenz des lebenden Probanden zugeordnet ist.

**Revendications**

1. Système d'analyse de forme d'onde intraveineuse (IV) périphérique (PIVA), comprenant :

un dispositif IV périphérique (110) configuré pour acquérir, d'une veine d'un sujet vivant, des signaux veineux périphériques ; et un dispositif de traitement (120) connecté en communication au dispositif IV périphérique, configuré pour :

recevoir (S210) les signaux veineux périphériques du dispositif IV périphérique ; et

réaliser (S220) une analyse spectrale sur le signal veineux périphérique pour obtenir un spectre de fréquence de pression veineuse périphérique ;

**caractérisé en ce que** le dispositif de traitement est configuré en outre pour :

réaliser (S230) une analyse statistique sur les amplitudes de pics du spectre de fréquence de pression veineuse périphérique pour déterminer un état de volume intravasculaire du sujet vivant en temps réel.

2. Système PIVA selon la revendication 1, dans lequel :

l'état de volume intravasculaire du sujet vivant indique une hypovolémie lorsque des baisses d'amplitude supérieures à un premier seuil sont détectées à partir des pics du spectre de fréquence de pression veineuse périphérique ; et

l'état de volume intravasculaire du sujet vivant indique une hypervolémie lorsque des réductions d'amplitude supérieures à un deuxième seuil sont détectées à partir des pics du spectre de fréquence de pression veineuse périphérique.

3. Système PIVA selon la revendication 1, appliqué au sujet vivant pendant une réanimation du sujet vivant, dans lequel :

l'état de volume intravasculaire du sujet vivant indique un retour d'euvolémie à partir d'un état hypovolémique lorsque le sujet vivant est déterminé comme étant dans l'état hypovolémique à une période antérieure, et des augmentations d'amplitude supérieures à un troisième seuil sont détectées à partir des pics du spectre de fréquence de pression veineuse périphérique ; et

l'état de volume intravasculaire du sujet vivant indique une surréanimation lorsque le sujet vivant est déterminé comme étant dans un état euvolémique à la période antérieure, et des augmentations d'amplitude supérieures à un quatrième seuil sont détectées à partir des pics du spectre de fréquence de pression veineuse périphérique ; dans lequel le dispositif de traitement est configuré en outre pour détecter l'efficacité du traitement et le retour à l'euvolémie chez le sujet vivant sur la base de l'état de volume intravasculaire du sujet vivant.

4. Système PIVA selon la revendication 1, dans lequel le dispositif de traitement est connecté au dispositif IV périphérique de manière communicative par l'intermédiaire d'une connexion sans fil, et le dispositif IV périphérique comprend :

un cathéter IV périphérique inséré dans la veine du sujet vivant ; et

un dispositif de surveillance connecté au cathéter IV périphérique, configuré pour capturer et enregistrer les signaux veineux périphériques provenant du cathéter IV périphérique à une vitesse d'échantillonnage, dans lequel le dispositif de surveillance comprend un transducteur de pression connecté directement au cathéter IV périphérique, dans lequel les signaux veineux périphériques sont capturés et enregistrés par le transducteur de pression.

5. Système PIVA selon la revendication 1, comprenant en outre :

une pompe connectée au sujet vivant pour réaliser un échange de liquide par rapport au sujet vivant, dans lequel la pompe est une pompe de dialyse, une pompe de pontage cardiopulmonaire, une oxygénation par membrane extracorporelle (ECMO), ou une pompe à perfusion ; et

un mécanisme de commande de pompe connecté en communication au dispositif de traitement, configuré pour commander la pompe par arrêt intermittent de la pompe ou soustraire le signal de pompe lorsque le dispositif IV périphérique acquiert les signaux veineux périphériques, et redémarrage de la pompe lorsque le dispositif IV périphérique n'acquiert pas les signaux veineux périphériques ;

dans lequel le dispositif de traitement est configuré en outre pour envoyer un signal au mécanisme de commande de pompe pour notifier au mécanisme de commande de pompe de commander la pompe.

6. Système PIVA selon la revendication 2, configuré pour être utilisé sur le sujet vivant pendant une ultrafiltration/dialyse ou diurèse du sujet vivant, dans lequel le dispositif de traitement (120) est configuré en outre pour :

générer un message d'alerte lorsque l'état de volume intravasculaire du sujet vivant indique une hypovolémie.

7. Système PIVA selon la revendication 1, dans lequel l'analyse spectrale est une analyse spectrale transformée de Fourier rapide (FFT), dans lequel l'analyse statistique comprend :

l'obtention d'une pluralité de pics de référence $\{B_{N-1}\}$ sur un spectre de fréquence de pression veineuse périphérique de référence, dans lequel N est un entier positif, et la pluralité de pics de référence $\{B_{N-1}\}$ correspond respectivement à une pluralité de fréquences $\{F_0, F_1, ..., F_N\}$, de telle sorte que $B_{N-1}$ est une fonction de $F_{N-1}$ satisfaisant $B_{N-1} = B_{N-1} (F_{N-1})$, dans lequel $F_N$ est supérieur à $F_{N-1}$ ;

l'obtention d'une pluralité de pics $\{P_{N-1}\}$ sur le spectre de fréquence de pression veineuse périphérique, dans lequel la pluralité de pics $\{P_{N-1}\}$ correspondent à la pluralité de fréquences $\{F_0, F_1, ..., F_N\}$, de telle sorte que $P_{N-1}$ est une fonction de $F_{N-1}$ satisfaisant $P_{N-1} = P_{N-1} (F_{N-1})$ ; et

la détermination de l'état de volume intravasculaire du sujet vivant en temps réel par comparaison des amplitudes des pics $\{P_{N-1}\}$ à celui des pics de référence $\{B_{N-1}\}$ respectivement, dans lequel

la pluralité de pics $\{P_{N-1}\}$ comprend un premier pic $P_0$ correspondant à une première fréquence $F_0$ et un deuxième pic $P_1$ correspondant à une deuxième fréquence $F_1$ ;

le premier pic $P_0$ correspondant à la première fréquence $F_0$ est associé à une fréquence respiratoire du sujet vivant ; et

le deuxième pic $P_1$ correspondant à la deuxième fréquence $F_1$ est associé à une fréquence cardiaque du sujet vivant ;

dans lequel le spectre de fréquence de pression veineuse périphérique de référence est obtenu par :

acquisition des signaux veineux périphériques de la veine du sujet vivant à une période antérieure ; et
traitement des signaux veineux périphériques acquis à la période antérieure par l'analyse FFT spectrale pour obtenir le spectre de fréquence de pression veineuse périphérique de référence.

8. Système PIVA selon la revendication 1, pour déterminer une hypovolémie, hypervolémie et le tonus vasculaire du sujet vivant sur la base de l'état de volume intravasculaire du sujet vivant, dans lequel

le dispositif IV périphérique (110) est configuré en outre pour acquérir (S210), en continu pendant une période allant de $T_0$ à $T_2$, les signaux veineux périphériques provenant de la veine du sujet vivant, dans lequel la période est divisée en une première période allant de $T_0$ à $T_1$, et une deuxième période allant de $T_1$ à $T_2$ ; et
le dispositif de traitement (120) est configuré en outre pour :

traiter les signaux veineux périphériques acquis à la première période pour obtenir une référence du spectre de fréquence de pression veineuse périphérique ;
dans lequel la réalisation de l'analyse statistique comprend :

l'obtention d'une pluralité de pics de référence $\{B_{N-1}\}$ sur le spectre de fréquence de pression veineuse périphérique de référence, dans lequel N est un entier positif, et la pluralité de pics de référence $\{B_{N-1}\}$ correspondent respectivement à une pluralité de fréquences $\{F_0, F_1, ..., F_N\}$, de telle sorte que $B_{N-1}$ est une fonction de $F_{N-1}$ satisfaisant $B_{N-1} = B_{N-1} (F_{N-1})$, dans lequel $F_N$ est supérieur à $F_{N-1}$ ;
le traitement (S220) des signaux veineux périphériques acquis à la deuxième période pour obtenir un spectre de fréquence de pression veineuse périphérique ;
l'obtention d'une pluralité de pics $\{P_{N-1}\}$ sur le spectre de fréquence de pression veineuse périphérique, dans lequel la pluralité de pics $\{P_{N-1}\}$ correspondent à la pluralité de fréquences $\{F_0, F_1, ..., F_N\}$, de telle sorte que $P_{N-1}$ est une fonction de $F_{N-1}$ satisfaisant $P_{N-1} = P_{N-1} (F_{N-1})$ ; et
la détermination (S230, S240) de l'état de volume intravasculaire du sujet vivant à la deuxième période par comparaison des amplitudes des pics $\{P_{N-1}\}$ à celui des pics de référence $\{B_{N-1}\}$ respectivement, dans lequel l'état de volume intravasculaire du sujet vivant à la deuxième période indique une hypovolémie ou hypervolémie (S250) lorsque des modifications d'amplitude supérieures à un seuil sont détectées des pics de référence $\{B_{N-1}\}$ aux pics $\{P_{N-1}\}$.

9. Système selon la revendication 8, dans lequel :

l'état de volume intravasculaire du sujet vivant à la deuxième période indique une hypovolémie lorsque des baisses d'amplitude sont détectées comme étant supérieures à un premier seuil allant des pics de référence $\{B_{N-1}\}$ aux pics $\{P_{N-1}\}$ ; et
l'état de volume intravasculaire du sujet vivant à la deuxième période indique une hypervolémie lorsque des augmentations d'amplitude supérieures à un deuxième seuil sont détectées des pics de référence $\{B_{N-1}\}$ aux pics $\{P_{N-1}\}$.

10. Système selon la revendication 8, configuré pour être utilisé sur le sujet vivant pendant une réanimation du sujet vivant, dans lequel :

l'état de volume intravasculaire du sujet vivant à la deuxième période indique un retour d'euvolémie à partir d'un état hypovolémique lorsque le sujet vivant est déterminé comme étant dans l'état hypovolémique à une première période, et des augmentations d'amplitude supérieures à un troisième seuil sont détectées des pics de référence $\{B_{N-1}\}$ aux pics $\{P_{N-1}\}$ ; et

l'état de volume intravasculaire du sujet vivant à la deuxième période indique une surréanimation lorsque le sujet vivant est déterminé comme étant dans un état euvolémique à la période antérieure, et des augmentations d'amplitude supérieures à un quatrième seuil sont détectées des pics de référence $\{B_{N-1}\}$ aux pics $\{P_{N-1}\}$.

11. Système selon la revendication 8, dans lequel :

les signaux veineux périphériques sont traités par une analyse spectrale transformée de Fourier rapide (FFT) pour obtenir le spectre de fréquence de pression veineuse périphérique de référence et le spectre de fréquence de pression veineuse périphérique, respectivement ;

la pluralité de pics $\{P_{N-1}\}$ comprend un premier pic $P_0$ correspondant à une première fréquence $F_0$ et un deuxième pic $P_1$ correspondant à une deuxième fréquence $F_1$ ;

le premier pic $P_0$ correspondant à la première fréquence $F_0$ est associé à une fréquence respiratoire du sujet vivant ; et

le deuxième pic $P_1$ correspondant à la deuxième fréquence $F_1$ est associé à une fréquence cardiaque du sujet vivant.

EP 3 190 960 B1

100

110

120

130

Peripheral IV Device

Processing Device

FIG. 1

S210

Acquiring, from the vein of the living subject, the peripheral venous signals

S220

Performing spectral FFT analysis on the peripheral venous signals acquired obtain a peripheral venous pressure frequency spectrum

S230

Performing a statistical analysis on amplitudes of peaks of the peripheral venous pressure frequency spectrum to determine the blood volume status of the living subject in real time

S260

No hypovolemia or hypervolemia

No

S240

Significant amplitude change detected?

Yes

S250

hypovolemia or hypervolemia is detected

FIG. 2

FIG. 3

FIG. 4A

FIG. 4B

FIG. 5A

FIG. 5B

**(A)**

**Hypovolemia**

**(B)**

**Hypervolemia**

F₁ (Linear Correlation)
Shock Index
HR
MAP

FIG. 6

EP 3 190 960 B1

Table 1. Patient Demographics.

| Patient number | Age | Gender | Preoperative Diagnosis | Surgery | Wt. (kg) | BSA | Diabetes | Creatinine | Preoperative Hgb | Ejection Fraction | RV dysfunction | PIV size (gauge) | Baseline PAP (mean) |
|---|---|---|---|---|---|---|---|---|---|---|---|---|---|
| 1 | 70 | F | Severe MR | MVR | 85 | 1.9 | Yes | 1.52 | 12.7 | 45-50 | None | 18 | 34/12 (20) |
| 2 | 63 | M | Severe MR, CAD | MVR | 93 | 2.08 | Yes | 2.01 | 12.3 | 15-20 | None | 18 | 31/14 (20) |
| 3 | 54 | M | Severe MR | MV repair | 96 | 2.21 | No | 1.2 | 14.4 | >55 | None | 18 | 22/15 (17) |
| 4 | 85 | M | Severe AS, CAD | AVR, CABG | 89 | 2.1 | Yes | 0.88 | 12.5 | 30-40 | None | 18 | 25/17 (20) |
| 5 | 33 | M | Severe AI | AVR | 73 | 1.9 | No | 0.82 | 15.4 | >55 | None | 16 | 19/11 (14) |
| 6 | 68 | M | Severe AS | AVR | 98 | 2.25 | No | 1.79 | 13.5 | 35-44 | None | 18 | 18/10 (14) |
| 7 | 74 | F | CAD | CABG | 92 | 2.07 | No | 1.07 | 9.8 | >55 | None | 18 | 31/15 (21) |
| 8 | 71 | F | Severe MR, moderate TR | MVR, TV repair | 71 | 1.78 | No | 0.76 | 14.2 | 45-55 | None | 18 | 32/20 (19) |
| 9 | 76 | M | Severe MR | MV repair | 90 | 2.08 | No | 0.83 | 14.2 | >55 | None | 18 | 44/20 (23) |
| 10 | 58 | M | Chronic PE | Pulmonary endarterectomy | 80 | 1.94 | No | 1.15 | 13.9 | >55 | Mild | 16 | 115/42 (68) |
| 11 | 57 | M | Aortic dissection | Aortic dissection repair | 88 | 2.11 | No | 0.79 | 12.8 | >55 | None | 18 | 46/22 (15) |
| 12 | 77 | M | Severe MR | MVR | 103 | 2.27 | Yes | 1.28 | 15.6 | >55 | None | 18 | 68/32 (44) |

Abbreviations: AI – Aortic insufficiency, AS – Aortic stenosis, AVR – aortic valve replacement, BSA – body surface area, CABG – Coronary artery bypass grafting, CAD – Coronary artery disease, Hgb – hemoglobin, MR – mitral regurgitation, MV – mitral valve, MVR – mitral valve replacement, PAP – pulmonary artery pressure, PE – Pulmonary embolus, PIV – peripheral intravenous catheter, RV – Right ventricle, TR – tricuspid regurgitation, TV – tricuspid valve, Wt. - weight

## FIG. 7

FIG. 8

FIG. 9

FIG. 10A

| Variable | AUC | SE | 95% CI |
|---|---|---|---|
| f1 | 0.900 | 0.0722 | 0.667 to 0.990 |
| dpap | 0.619 | 0.137 | 0.365 to 0.833 |
| hr | 0.544 | 0.152 | 0.297 to 0.775 |
| map | 0.512 | 0.155 | 0.271 to 0.750 |

FIG. 10B

FIG. 11A

FIG. 11B

FIG. 12

FIG. 13

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 62049829
- US 85350415
- US 20100191128 A1

**Non-patent literature cited in the description**

- **WILSON, M. ; D.P. DAVIS ; R. COIMBRA.** Diagnosis and monitoring of hemorrhagic shock during the initial resuscitation of multiple trauma patients: a review. *The Journal of Emergency Medicine,* 2003, vol. 24 (4), 413-422
- **PAYEN, D. et al.** A positive fluid balance is associated with a worse outcome in patients with acute renal failure. *Crit Care,* 2008, vol. 12 (3), R74
- **WIEDEMANN, H.P. et al.** Comparison of two fluid-management strategies in acute lung injury. *N Engl J Med,* 2006, vol. 354 (24), 2564-75
- **BOUCHARD, J. et al.** Fluid accumulation, survival and recovery of kidney function in critically illpatients with acute kidney injury. *Kidney Int,* 2009, vol. 76 (4), 422-7
- **EDELMAN, D.A. et al.** Post-traumatic hypotension: should systolic blood pressure of 90-109 mmHg be included?. *Shock,* 2007, vol. 27 (2), 134-8
- **KUMAR, A. et al.** Pulmonary artery occlusion pressure and central venous pressure fail to predict ventricular filling volume, cardiac performance, or the response to volume infusion in normal subjects. *Crit Care Med,* 2004, vol. 32 (3), 691-9
- **MERMEL, L.** Infections related to central venous catheters in US intensive-care units. *Lancet,* 2003, vol. 361 (9368), 1562
- **GARLAND, A. ; A.F. CONNORS.** Indwelling arterial catheters in the intensive care unit: necessary and beneficial, or a harmful crutch?. *Am J Respir Crit Care Med,* 2010, vol. 182 (2), 133-4
- **DOMINO, K.B. et al.** Injuries and liability related to central vascular catheters: a closed claims analysis. *Anesthesiology,* 2004, vol. 100 (6), 1411-1418
- **BENDJELID, K. ; J. ROMAND.** Fluid responsiveness in mechanically ventilated patients: a review of indices used in intensive care. *Intensive Care Medicine,* 2003, vol. 29 (3), 352-360
- **EYRE, L. ; A. BREEN.** Optimal volaemic status and predicting fluid responsiveness. *Continuing Education in Anaesthesia, Critical Care & Pain,* 2010, vol. 10 (2), 59-62
- **MARIK, P.** Hemodynamic Parameters to Guide Fluid Therapy. *Transfusion Alternatives in Transfusion Medicine,* 2010, vol. 11 (3), 102-112
- **CHARALAMBOUS, C. et al.** Comparison of peripheral and central venous pressures in critically Illpatients. *Anaesth Intensive Care,* 2003, vol. 31 (1), 34-9
- **CHARALAMBOUS, C. et al.** Comparison of peripheral and central venous pressures in critically illpatients. *Anaesthesia and Intensive Care,* 2003, vol. 31 (1), 34-39
- **PIKWER, A. et al.** Fluid balance monitoring by cuff-occluded rate of rise of peripheral venous pressure in haemodialysis patients. *Anaesthesia,* 2012, vol. 67 (8), 894-8
- **ALIAN, A.A. et al.** Impact of lower body negative pressure induced hypovolemia on peripheral venous pressure waveform parameters in healthy volunteers. *Physiol Meas,* 2014, vol. 35 (7), 1509-20
- **SILESHI, B. et al.** Peripheral venous waveform analysis for detecting early hemorrhage: a pilot study. *Intensive Care Med,* 2015, vol. 41 (6), 1147-8
- **KUTCHER, M.E. et al.** A paradigm shift in trauma resuscitation: evaluation of evolving massive transfusion practices. *JAMA Surg,* 2013, vol. 148 (9), 834-40
- **HUANG, Q. et al.** Fluid volume overload negatively influences delayed primary facial closure in open abdomen management. *J Surg Res,* 2014, vol. 187 (1), 122-7
- Goal-Directed Resuscitation for Patients with Early Septic Shock. *N Engl J Med,* 2014
- **BLOW, O. et al.** The golden hour and the silver day: detection and correction of occult hypoperfusion within 24 hours improves outcome from major trauma. *J Trauma,* 1999, vol. 47 (5), 964-9
- **DESEBBE, O. ; M. CANNESSON.** Using ventilation-induced plethysmographic variations to optimize patient fluid status. *Curr Opin Anaesthesiol,* 2008, vol. 21 (6), 772-8

- **DE BACKER, D. et al.** Pulse pressure variations to predict fluid responsiveness: influence of tidal volume. *Intensive Care Med,* 2005, vol. 31 (4), 517-23
- **AULER, J.O., JR. et al.** Online monitoring of pulse pressure variation to guide fluid therapy after cardiac surgery. *Anesth Analg,* 2008, vol. 106 (4), 1201-6
- **BERKENSTADT, H. et al.** Pulse pressure and stroke volume variations during severe haemorrhage in ventilated dogs. *Br J Anaesth,* 2005, vol. 94 (6), 721-6
- **GRAHAM, M.R. ; K. MCCREA ; L.G. GIRLING.** Pulse pressure variability during hemorrhage and reinfusion in piglets: effects of age and tidal volume. *Can J Anaesth,* 2014, vol. 61 (6), 533-42
- **YANG, X. ; B. DU.** Does pulse pressure variation predict fluid responsiveness in critically ill patients? A systematic review and meta-analysis. *Crit Care,* 2014, vol. 18 (6), 650
- **OLIVEIRA-COSTA, C.D. et al.** Pulse pressure variation and prediction of fluid responsiveness in patients ventilated with low tidal volumes. *Clinics (Sao Paulo),* 2012, vol. 67 (7), 773-8
- **MARIK, P.E. ; X. MONNET ; J.L. TEBOUL.** Hemodynamic parameters to guide fluid therapy. *Ann Intensive Care,* 2011, vol. 1 (1), 1
- **HEENEN, S. ; D. DE BACKER ; J.L. VINCENT.** How can the response to volume expansion in patients with spontaneous respiratory movements be predicted?. *Crit Care,* 2006, vol. 10 (4), R102
- **SILESHI, B. et al.** Peripheral venous waveform analysis for detecting early hemorrhage: a pilot study. *Intensive Care Med,* 2015
- **CANNON, C.M. et al.** Utility of the shock index in predicting mortality in traumatically injured patients. *J Trauma,* 2009, vol. 67 (6), 1426-30
- **HOLTE, K. ; N. SHARROCK ; H. KEHLET.** Pathophysiology and clinical implications of perioperative fluid excess. *British Journal of Anaesthesia,* 2002, vol. 89 (4), 622-632
- **MABRY, R.L. et al.** Impact of critical care-trained flight paramedics on casualty survival during helicopter evacuation in the current war in Afghanistan. *J Trauma Acute Care Surg,* 2012, vol. 73, S32-7
- **PARKS, J.K. et al.** Systemic hypotension is a late marker of shock after trauma: a validation study of Advanced Trauma Life Support principles in a large national sample. *The American Journal of Surgery,* 2006, vol. 192 (6), 727-731
- **VINCENT, J.L. et al.** Clinical review: Update on hemodynamic monitoring--a consensus of 16. *Crit Care,* 2011, vol. 15 (4), 229
- **WHEELER, A.P. et al.** Pulmonary-artery versus central venous catheter to guide treatment of acute lung injury. *N Engl J Med,* 2006, vol. 354 (21), 2213-24

- **BUHRE, W. et al.** Changes in central venous pressure and pulmonary capillary wedge pressure do not indicate changes in right and left heart volume in patients undergoing coronary artery bypass surgery. *Eur J Anaesthesiol,* 1999, vol. 16 (1), 11-7
- **HOLCOMB JB ; MCMULLIN NR ; PEARSE L et al.** Causes of death in U.S. Special Operations Forces in the global war on terrorism: 2001-2004. *Annals of surgery,* 2007, vol. 245, 986-91
- **EASTRIDGE BJ ; HARDIN M ; CANTRELL J et al.** Died of wounds on the battlefield: causation and implications for improving combat casualty care. *The Journal of trauma,* 2011, vol. 71, S4-8
- **SWAROOP M ; STRAUS DC ; AGUBUZU O ; ESPOSITO TJ ; SCHERMER CR ; CRANDALL ML.** Pre-hospital transport times and survival for Hypotensive patients with penetrating thoracic trauma. *Journal of emergencies, trauma, and shock,* 2013, vol. 6, 16-20
- **RISKIN DJ ; TSAI TC ; RISKIN L et al.** Massive transfusion protocols: the role of aggressive resuscitation versus product ratio in mortality reduction. *Journal of the American College of Surgeons,* 2009, vol. 209, 198-205
- **DUKE MD ; GUIDRY C ; GUICE J et al.** Restrictive fluid resuscitation in combination with damage control resuscitation: time for adaptation. *The journal of trauma and acute care surgery,* 2012, vol. 73, 674-8
- **PALADINO L ; SINERT R ; WALLACE D ; ANDERSON T ; YADAV K ; ZEHTABCHI S.** The utility of base deficit and arterial lactate in differentiating major from minor injury in trauma patients with normal vital signs. *Resuscitation,* 2008, vol. 77, 363-8
- **CONVERTINO VA.** Blood pressure measurement for accurate assessment of patient status in emergency medical settings. *Aviation, space, and environmental medicine,* 2012, vol. 83, 614-9
- **CONVERTINO VA ; RYAN KL ; RICKARDS CA et al.** Physiological and medical monitoring for en route care of combat casualties. *The Journal of trauma,* 2008, vol. 64, 342-53
- **GUTIERREZ G ; REINES HD ; WULF-GUTIERREZ ME.** Clinical review: hemorrhagic shock. *Critical care,* 2004, vol. 8, 373-81
- **COCCHI MN ; KIMLIN E ; WALSH M ; DONNINO MW.** Identification and resuscitation of the trauma patient in shock. *Emergency medicine clinics of North America,* 2007, vol. 25, 623-42
- **MARIK PE ; CAVALLAZZI R ; VASU T ; HIRANI A.** Dynamic changes in arterial waveform derived variables and fluid responsiveness in mechanically ventilated patients: a systematic review of the literature. *Critical care medicine,* 2009, vol. 37, 2642-7
- **TEBOUL JL ; MONNET X.** Prediction of volume responsiveness in critically ill patients with spontaneous breathing activity. *Current opinion in critical care,* 2008, vol. 14, 334-9